# EUROPÄISCHE PATENTSCHRIFT

(11) **EP 2 783 753 B1**
(45) Veröffentlichungstag und Bekanntmachung des Hinweises auf die Patenterteilung: **22.02.2017**
(21) Anmeldenummer: 13001617.3
(22) Anmeldetag: 28.03.2013
(51) Int. Cl.: A61M 5/31, A61M 5/315, B01L 3/02

(54) **DISPENSER ZUM BETÄTIGEN EINER SPRITZE**
Dispenser for actuating an injection syringe
Distributeur destiné à actionner une seringue

(43) Veröffentlichungstag der Anmeldung: 01.10.2014
(73) Patentinhaber: EPPENDORF AG, 22339 Hamburg (DE)
(72) Erfinder: Reichmuth, Burkhardt, 20251 Hamburg (DE); Sattler, Jörg, 20251 Hamburg (DE)
(74) Vertreter: Hauck Patentanwaltspartnerschaft mbB

(56) Entgegenhaltungen:
- EP-A2- 1 110 612
- EP-B1- 0 656 229
- DE-A1- 19 615 918
- DE-U1-202010 010 942
- US-A- 3 977 574
- US-A1- 2004 210 200

## Beschreibung

Die Erfindung bezieht sich auf einen Dispenser zum Betätigen einer Spritze.

Die hier betrachteten Dispenser zum Betätigen einer Spritze dienen dazu, in die Spritze Flüssigkeit aufzunehmen und die aufgenommene Flüssigkeit in mehreren Schritten abzugeben. Sie werden auch als Repetierpipetten oder als Pipetten bezeichnet. Diese Dispenser haben am unteren Ende eines stangenförmigen Gehäuses eine Aufnahme für einen Flansch eines Zylinders der Spritze und in dem Gehäuse einen verlagerbaren Aufnahmekörper mit einer Aufnahme für den oberen Endbereich einer Kolbenstange eines Kolbens der Spritze. Die Spritze ist mit dem Flansch und dem Endbereich der Kolbenstange durch axial gerichtete Öffnungen der Aufnahme einsetzbar. Der Flansch und der Endbereich werden in den Aufnahmen von Mitteln zum lösbaren Halten gehalten, die beispielsweise als verfederte Greifhebel ausgebildet sind. Ferner weist der Dispenser Mittel zum Verlagern des Aufnahmekörpers auf, die ein teilweises Herausziehen des Kolbens aus dem Zylinder für ein Einsaugen von Flüssigkeit in die Spritze und ein schrittweises Eindrücken des Kolbens in den Zylinder für die schrittweise Abgabe von Flüssigkeit ermöglichen.

Die DE 29 26 691 C2 und die US 4,406,170 A beschreiben Mittel zum Verlagern des Aufnahmekörpers im Gehäuse. Diese umfassen einen mit dem Aufnahmekörper verbundenen und durch einen geradlinigen Schlitz aus dem Gehäuse herausstehenden Aufzugshebel zum Aufziehen von Flüssigkeit in die Spritze durch Verlagern des Aufnahmekörpers von der Aufnahme am unteren Ende des Gehäuses weg. Zudem umfassen sie einen hin- und herbewegbaren Dosierhebel zur schrittweisen Kolbenvorbewegung. An dem Dosierhebel ist eine schwenkbare Klinke gelagert und eine mit dem Aufnahmekörper verbundene Zahnstange ist im Schwenkbereich der Klinke angeordnet. Wenn der Dosierhebel nach unten geschwenkt wird, greift die Klinke in die Zahnstange ein und bewegt diese einen Schritt nach unten. Bei der Bewegung des Dosierhebels nach oben kommt die Klinke außer Eingriff mit der Zahnung. Danach kann durch erneutes Bewegen des Dosierhebels nach unten ein weiterer Dosierschritt ausgeführt werden.

Zur Einstellung der Dosiermenge (Dispensiervolumen), die bei einem Dosierschritt abgegeben wird, deckt eine einstellbar verschiebbare Abdeckung die Zahnung auf der Zahnstange mehr oder weniger ab. Hierdurch wird der Eingriff der Klinke in die Zahnstange beim Schwenken des Dosierhebels begrenzt. Die verschiebbare Abdeckung ist mittels eines Drehknopfes verlagerbar, der am Gehäuse des Dispensers gelagert ist. Der Drehknopf weist eine exzentrische Führungskurve auf, in die ein Führungszapfen der verschiebbaren Abdeckung eingreift. Das Ausmaß der Überdeckung der Zahnung durch die Abdeckung hängt von der Stellung des Drehknopfes ab. Dementsprechend ist mittels des Drehknopfes das Ausmaß der Verlagerung der Zahnstange und damit die bei einem Dosierschritt abgegebene Dosiermenge einstellbar.

Die Zahnstange hat einen hochragenden Ansatz, durch den in vorgeschobener Lage des Kolbens die Abdeckung von der Zahnstange weg verlagerbar ist, sodass sie die Klinke daran hindert, in die nicht abgedeckten Zähne der Zahnstange einzugreifen. Hierdurch wird eine Resthubsperre verwirklicht, die verhindert, dass eine Restmenge aus der Spritze abgegeben wird, die kleiner als die bei einem Dosierschritt abzugebende Dosiermenge ist.

Weiterentwicklungen der Mittel zum lösbaren Halten der Spritze sind in der EP 0 656 229 B1 und US 5,620,660 A beschrieben.

Die EP 1 724 020 B1 und die US 7,731,908 B2 beschreiben eine Weiterentwicklung der Halteeinrichtungen, die ein Lösen der Spritze von der Pipette durch Einhandbedienung ermöglichen. Bei dieser Pipette muss der Benutzer zum Lösen der Spritze einen Auslöser betätigen, der zusätzlich zum Dosierhebel vorhanden ist. Die EP 2 033 712 A1 und die US 2009/139351 A1 beschreiben eine Weiterentwicklung, mit der die Spritze nach dem letzten Dispensierschritt durch erneutes Betätigen des Dosierhebels von der Pipette gelöst werden kann. Hierdurch wird die Einhandbedienung erleichtert, da der Anwender nicht vom Dosierhebel auf einen Auslöser umgreifen muss.

Die EP 0 657 216 B1 und US 5,620,661A beschreiben eine derartige Pipette mit einem Sensor zum Abtasten von Erhebungen und Vertiefungen auf dem Spritzenflansch der Spritzen und entsprechend ausgestaltete Spritzen. Der Sensor dient dazu, die Größe der eingesetzten Spritze zu ermitteln. Auf der Grundlage der eingestellten Schrittweite ermittelt eine Elektronik die bei jedem Abgabeschritt abgegebene Flüssigkeitsmenge und zeigt diese auf einem Display an.

Weiterentwicklungen der Mittel zum Verlagern des Aufnahmekörpers sind in der DE 44 37 716 C2, EP 0 697 439 B1 und US 5,591,408A beschrieben. Gemäß EP 0 679 439 B1 und US 5,591,408A hat eine Repetierpipette eine Konstantschritteinrichtung, welche die Weite des ersten Schrittes zum Verschieben des Aufnahmekörpers für die Kolbenstange zur Aufnahme für den Spritzenflansch hin auf einen konstanten Wert festlegt, der unabhängig von der Einstellung der nachfolgenden Schrittweiten ist. Hierbei wird nach dem Aufziehen von Flüssigkeit zu Beginn des Verlagerns des Aufnahmekörpers nach unten ein konstanter Umkehrhub ausgeführt, der ein die Dosiergenauigkeit beeinträchtigendes Spiel zwischen Dispenser und Spritze überwindet.

Gemäß EP 2 033 712 A1 und US 2009/139351 A1 umfassen die Kolbenstelleinrichtungen anstatt einer Zahnstange eine Gewindespindel, die fest mit dem Aufnahmekörper verbunden ist. Hierfür hat der Aufnahmekörper eine Bohrung, welche das untere Ende der Gewindespindel aufnimmt. Der Aufnahmekörper hat an einander diametral gegenüberliegenden Seiten Durchbrüche. In den Durchbrüchen sind am Aufnahmekörper auf Schwenkachsen Kolbengreifhebel gelagert. Die Kolbengreifhebel können einen Kolbenbund am äußeren Ende des Spritzenkolbens hintergreifen. Dafür haben sie oberhalb der Schwenkachsen einen etwa keilförmigen Greifarm. Die Kolbengreifhebel sind durch Schenkelfedern in Richtung auf eine Position vorgespannt, in der sie den Kolbenbund hintergreifen.

Bei einer praktischen Ausführung besteht die Gewindespindel aus Metall und der Aufnahmekörper aus Kunststoff. Die Gewindespindel hat am vorderen Ende zwei Nuten. Die Montage der Gewindespindel mit dem Aufnahmekörper erfolgt, indem von unten zwei Schrauben in den Aufnahmekörper eingeschraubt werden, die in die Nuten der eingesetzten Gewindespindel eingreifen und diese im Aufnahmekörper fixieren. Bei der Montage muss die Drehstellung der Gewindespindel bezüglich des Aufnahmekörpers eingestellt werden, damit der Dispenser mit der gewünschten Genauigkeit arbeitet. Nachteilig bei diesem Dispenser ist das relativ hohe Gewicht und der hohe Montageaufwand von Gewindestange, Aufnahmekörper und Kolbengreifhebeln.

Die EP 2 415 525 A2 beschreibt eine Pipette für ein Pipettensystem aus einer Pipette und einer Spritze. Im Pipettengehäuse der Pipette befindet sich eine Kolbenstelleinrichtung mit einer axial verschiebbaren Zahnstange mit einer Verzahnung. Die Zahnstange ist mit dem Kolbenkopf einer eingesetzten Spritze gekoppelt. Die Zahnstange ist mehrteilig ausgeführt und besteht zumindest aus einem Träger und einem Verzahnungsteil. Der Träger weist eine Aufnahme für das Verzahnungsteil auf und das Verzahnungsteil seinerseits trägt die Verzahnung. Aufgrund dieser Zweiteiligkeit der Zahnstange kann man den Träger einerseits und das Verzahnungsteil andererseits aus unterschiedlichen Materialien ausführen, die den jeweiligen speziellen Anforderungen angepasst sind.

Bei einem Ausführungsbeispiel ist eine halbzylindrische Aufnahme für den Kolbenkopf einteilig mit dem Träger der Zahnstange ausgeführt, beispielsweise als einstückiges Spritzgussteil aus Kunststoff. An der Aufnahme sind ein halbzylindrisches Klemmelement und eine Hebelmechanik gelagert, mit der ein Kolbenkopf in der Aufnahme fixierbar ist.

Nachteilig bei dieser Konstruktion ist, dass die Toleranzen der Bauteile einander überlagern. Damit die Pipette mit der gewünschten Genauigkeit arbeitet, müssen die Toleranzen der einzelnen Bauteile gering gehalten bzw. aufeinander abgestimmt werden. Temperaturschwankungen können aufgrund der unterschiedlichen Wärmedehnung der Materialien die Präzision der Pipette verändern. Die Montage von Träger, Verzahnung und Hebelmechanik ist aufwendig. Zudem ist die Konstruktion raumgreifend und schwer. Schließlich können sich insbesondere an den Fugen zwischen den verschiedenen Bauteilen Verschmutzungen ansammeln. Verschmutzungen können den Eingriff der Klinke in die Zahnung verhindern und die Präzision der Dosierung beeinträchtigen. Schmutz kann durch die Öffnung für die Spritze in das Gehäuse eintreten. Ferner kann sich Abrieb aus der Mechanik an den Fugen festsetzen.

US 2004/210200 A1 beschreibt einen Dispenser mit einer Spritze, die in einem Gehäuse des Dispensers angeordnet ist, sodass nur die Nadel nach außen heraussteht. Der Spritzenkörper wird vom Gehäuse aufgenommen und der Kolben ist mit dem vorderen Ende einer Zahnstange gekoppelt. Hierfür ist das untere Ende der Zahnstange in eine Vertiefung im oberen Ende des Kolbens eingeklemmt.

US 3,977,574 beschreibt eine Pipette mit einem fest eingebauten Kolben zum Ansaugen und Ausstoßen von Flüssigkeit aus einer Pipettenspitze. Die Pipettenspitze ist ein austauschbares Einmalteil.

DE 196 15 918 A1 beschreibt ebenfalls Pipetten, die dauerhaft in einem Gehäuse einen Zylinder und einen darin verschieblich angeordneten Kolben haben. Ein Austauschteil in Form einer Pipettenspitze ist auf das untere Ende des Zylinders aufklemmbar. Mittels eines Abwerfers ist die Pipettenspitze vom Ansatz abdrückbar.

Davon ausgehend liegt der Erfindung die Aufgabe zugrunde, einen Dispenser zur Verfügung zu stellen, der bei geringerem Montageaufwand sowie reduziertem Gewicht und Raumbedarf eine erhöhte Präzision begünstigt.

Die Aufgabe wird durch einen Dispenser mit den Merkmalen von Anspruch 1 gelöst. Vorteilhafte Ausgestaltungen des Dispensers sind in Unteransprüchen angegeben.

Der erfindungsgemäße Dispenser zum Betätigen einer Spritze für das Aufnehmen und schrittweise Abgeben von Flüssigkeit hat
- ein stangenförmiges Gehäuse,
- eine erste Aufnahme mit einer ersten Öffnung am unteren Ende des Gehäuses zum Einsetzen eines ersten Befestigungsabschnitts am oberen Rand eines Zylinders der Spritze,
- einen im Gehäuse angeordneten Aufnahmekörper mit einer zweiten Aufnahme und einer zweiten Öffnung am unteren Ende zum Einsetzen eines zweiten Befestigungsabschnitts an einem Kolben der Spritze,
- erste Mittel zum lösbaren Halten des ersten Befestigungsabschnitts in der ersten Aufnahme,
- zweite Mittel zum lösbaren Halten des zweiten Befestigungsabschnittes in der zweiten Aufnahme,
- erste Mittel zum Verlagern des Aufnahmekörpers im Gehäuse nach oben,
- zweite Mittel zum schrittweisen Verlagern des Aufnahmekörpers im Gehäuse nach unten mit
- einem aus dem Gehäuse herausragenden Betätigungselement zum Durchführen einzelner Schritte,
- einer im Gehäuse angeordnete und mit dem Aufnahmekörper verbundenen Zahnstange mit einer Zahnung und
- einer im Gehäuse am Betätigungselement schwenkbar gelagerten Klinke, die bei einer Verlagerung des Betätigungselements nach unten mit der Zahnung der Zahnstange in Eingriff kommt und diese mitnimmt und bei Verlagerung des Betätigungselements nach oben außer Eingriff mit der Zahnung kommt,
dadurch gekennzeichnet, dass
- die Zahnstange und der Aufnahmekörper einteilig aus Kunststoff hergestellt sind und
- die zweiten Mittel zum lösbaren Halten mindestens einen in einer seitlichen Öffnung des Aufnahmekörpers angeordneten und einteilig mit dem Aufnahmekörper aus elastischem Kunststoff hergestellten zweiten Greifhebel aufweisen.

Der erfindungsgemäße Dispenser weist eine Zahnstangen-Klinkeneinrichtung für die schrittweise Verlagerung des Aufnahmekörpers nach unten auf, damit dieser den Kolben einer Spritze entsprechend verlagert und die Spritze in mehreren Schritten bestimmte Dosiermengen abgibt. Die Besonderheit des Dispensers besteht darin, dass die Zahnstange und der Aufnahmekörper einteilig aus Kunststoff hergestellt sind. Durch die einteilige Ausführung von Zahnstange und Aufnahmekörper aus Kunststoff kann Gewicht und Raumbedarf eingespart werden. Der Aufwand für die Montage mehrerer Bauteile wird vermieden. Zudem wird die Präzision des Dispensers verbessert, weil die einteilige Ausbildung von Zahnstange und Aufnahmekörper geringe Toleranzen begünstigt und unterschiedliche Wärmedehnungen verschiedener Materialien vermeidet. Die einteilige Ausführung der Zahnstange hat zudem den Vorteil, dass Schmutzansammlungen vermieden werden, die sich bei einer Zahnstange aus einem Träger und einem zusätzlichen Verzahnungsteil an den Fugen ansammeln können.

Gemäß einer bevorzugten Ausgestaltung werden Zahnstange und Aufnahmekörper einteilig durch Spritzgießen aus Kunststoff hergestellt. Die Herstellung ist insbesondere durch ein Spritzgießwerkzeug mit zwei Formhälften mit einer parallel zur Zahnung ausgerichteten Trennebene und einem Kern zum Formen der zweiten Aufnahme des Aufnahmekörpers möglich.

Gemäß einer Ausgestaltung der Erfindung ist der Aufnahmekörper hohlzylindrisch und einteilig mit dem unteren Ende der Zahnstange verbunden. Diese Ausgestaltung ist für die Herstellung und Stabilität besonders vorteilhaft.

Gemäß einer bevorzugten Ausgestaltung ist der Aufnahmekörper ein Käfig, der am unteren Ende ein Ringelement und von dem Ringelement ausgehend parallel nach oben erstreckte Streben aufweist, die am oberen Ende mit dem unteren Ende der Zahnstange verbunden sind. Dies ist vorteilhaft für eine einfache und materialsparende Herstellung mit der erforderlichen Festigkeit.

Gemäß einer weiteren Ausgestaltung weist der Käfig eine vordere Strebe auf der Seite der Zahnung der Zahnstange und eine hintere Strebe auf der von der Zahnung abgewandten Seite der Zahnstange auf. Diese Ausgestaltung ist besonders vorteilhaft für die Herstellung mittels eines Spritzgießwerkzeugs mit zwei Formhälften.

Gemäß einer bevorzugten Ausgestaltung ist der Aufnahmekörper an der zweiten Öffnung trichterförmig nach unten erweitert. Gemäß einer weiteren Ausgestaltung weist das Ringelement des Käfigs die Erweiterung auf. Die Erweiterung erleichtert das Einführen des zweiten Befestigungsabschnittes einer Spritze in die zweite Aufnahme.

Die ersten Mittel zum lösbaren Halten sind bevorzugt erste Greifhebel. Insoweit wird Bezug genommen auf die Dokumente EP 0 656 229 B1 und US 5,620,660 A oder EP 1 724 120 B1 und US 7,731,908 B2, deren Inhalt hiermit in die vorliegende Anmeldung aufgenommen wird.

Die zweiten Mittel zum lösbaren Halten weisen mindestens einen in einer seitlichen Öffnung des Aufnahmekörpers angeordneten und einteilig mit dem Aufnahmekörper aus elastischem Kunststoff hergestellten zweiten Greifhebel auf. Vorzugsweise ist der Aufnahmekörper spritzgegossen und ist der zweite Greifhebel an den Aufnahmekörper angespritzt. Der zweite Greifhebel ist elastisch, sodass er hinter einem Bund oder in einer Nut des zweiten Befestigungsabschnitts des Kolbens verrastbar ist. Durch die einteilige Ausbildung des zweiten Greifhebels mit dem Aufnahmekörper wird Gewicht und Raumbedarf eingespart, Aufwand für weitere Bauteile vermieden, geringe Toleranzen begünstigt und die Präzision verbessert. Vorzugsweise weist der Aufnahmekörper mehrere, vorzugsweise zwei einteilig mit ihm verbundene zweite Greifhebel auf, die gleichmäßig um die zweite Aufnahme verteilt sind.

Gemäß einer bevorzugten Ausgestaltung ist der zweite Greifhebel einarmig, an einem Ende mit dem Aufnahmekörper verbunden, erstreckt sich parallel zur Zahnstange und weist in einem Abstand von seinem mit dem Aufnahmekörper verbundenen Ende einen nach innen vorstehenden Rastvorsprung auf. Der einarmige zweite Greifhebel ist unter einem Bund oder in einer Nut des zweiten Befestigungsabschnittes verrastbar. Die Verbindung des einarmigen zweiten Greifhebels an einem Ende mit dem Aufnahmekörper hat den Vorteil einer erhöhten Stabilität. Diese Ausgestaltung kann so ausgeführt werden, dass bei Einwirkung einer hohen Zugkraft auf den Spritzenkolben (z.B. beim Aufziehen einer hochviskosen Flüssigkeit in einer Spritze) der zweite Greifhebel selbsttätig öffnet und somit Überlastungen und Materialbruch vermieden werden können. Bei herkömmlichen Dispensern mit auf Stiften gelagerten zweiarmigen Hebeln kann eine solche Belastung zu einem verstärkten Festklemmen des zweiten Befestigungsabschnittes im Aufnahmekörper bis zum Herausbrechen der Hebel aus dem Aufnahmekörper führen.

Der zweite Griffhebel kann allein durch die Elastizität des verwendeten Kunststoffs den zweiten Befestigungsabschnitt im Aufnahmekörper festklemmen. Gemäß einer bevorzugten Ausgestaltung ist mindestens ein Federelement vorhanden, welches den zweiten Greifhebel in Richtung auf die zweite Aufnahme belastet. Das Federelement ist gemäß einer weiteren Ausgestaltung ein O-Ring, der unter Vorspannung an der Außenseite jedes zweiten Greifhebels anliegt.

Gemäß einer Ausgestaltung weist der Aufnahmekörper eine weitere seitliche Öffnung auf, stehen an beiden Längsseiten der weiteren seitlichen Öffnung
Lageraugen nach außen von dem Aufnahmekörper vor, ist ein weiterer zweiter Greifhebel auf einer Welle oder Achse in die weitere seitliche Öffnung hinein schwenkbar an den Lageraugen gelagert und weist der weitere zweite Greifhebel in einem Abstand von der Welle oder Achse einen nach innen vorstehenden Rastvorsprung auf. Der Dispenser kann ausschließlich auf einer Welle oder Achse schwenkbar gelagerte weitere zweite Greifhebel zum lösbaren Halten des zweiten Befestigungsabschnittes in der zweiten Aufnahme aufweisen. Bevorzugt weist der Dispenser sowohl einteilig mit dem Aufnahmekörper verbundene, als auch auf einer Welle oder Achse schwenkbar gelagerte zweite Greifhebel auf. Eine Kombination aus zwei einteilig mit dem Aufnahmekörper verbundenen und einem auf einer Welle oder Achse gelagerten zweiten Greifhebel ist für die Herstellung durch Spritzgießen besonders vorteilhaft und ermöglicht eine Dreipunktfixierung des zweiten Befestigungsabschnittes, die ein seitliches Ausweichen des zweiten Befestigungsabschnittes aus den zweiten Greifhebeln verhindert.

Gemäß einer bevorzugten Ausgestaltung ist der weitere zweite Greifhebel einarmig und an einem Ende auf einer Welle oder Achse an den Lageraugen gelagert. Die Lagerung des weiteren, zweiten Griffhebels an einem Ende hat den Vorteil einer erhöhten Stabilität. Diese Ausgestaltung kann so ausgeführt werden, dass der weitere zweite Greifhebel selbsttätig öffnet, wenn eine hohe Zugkraft auf den Spritzenkolben einwirkt, wodurch Überlastung und Materialbruch vermieden werden können.

Gemäß einer weiteren Ausgestaltung ist der zweite Greifhebel in einem Langloch zwischen den beiden Streben angeordnet und/oder ist der weitere zweite Greifhebel in einem weiteren Langloch in einer Strebe angeordnet. Diese Ausgestaltung ist für die Herstellung durch Spritzgießen besonders vorteilhaft. Ferner ist diese Ausgestaltung besonders vorteilhaft für eine raumsparende und leichte Ausführung, weil die zweiten Mittel zum lösbaren Halten entfernt von Zahnung und Aufzugshebel angeordnet sind.

Gemäß einer weiteren Ausgestaltung ist der zweite Greifhebel an seinem unteren Ende einteilig mit dem Aufnahmekörper verbunden und/oder ist der weitere zweite Greifhebel an seinem unteren Ende auf der Welle oder Achse gelagert. Gemäß einer weiteren Ausgestaltung ist der zweite Greifhebel an einem Ende mit dem Ringelement des Aufnahmekörpers verbunden. Gemäß einer weiteren bevorzugten Ausgestaltung sind die Lageraugen neben dem unteren Ende des weiteren Langlochs angeordnet.

Gemäß einer weiteren Ausgestaltung ist unten auf der Zahnstange und/oder oben auf den Streben ein Schieber in Längsrichtung der Zahnstange verschieblich angeordnet, der auf mindestens einer Seite eine Rampe aufweist, deren Abstand von der Zahnstange nach oben zunimmt, wobei der Schieber nach unten verlagerbar ist, sodass die Rampe das obere Ende des zweiten Greifhebels innen kontaktiert und bei weiterer Verlagerung des Schiebers nach unten den zweiten Greifhebel nach außen schwenkt. Der Schieber ermöglicht somit, den zweiten Befestigungsabschnitt einer Spritze vom Aufnahmekörper zu lösen. Der Schieber ist vorzugsweise mittels einer Abwurfeinrichtung betätigbar, die auch auf die ersten Mittel zum lösbaren Halten einwirkt, um gleichzeitig den ersten Befestigungsabschnitt der Spritze zu lösen. Die Abwurfeinrichtung kann einen gesonderten Auslöser haben oder mit einem Betätigungsorgan gekoppelt sein. In dieser Hinsicht wird Bezug genommen auf die eingangs erwähnten Dokumente EP 1 724 020 B1 und US 7,731,908 B2 oder EP 2 033 712 A1 und US 2009/139351 A1, deren Inhalt hiermit in die vorliegende Anmeldung aufgenommen wird.

Gemäß einer bevorzugten Ausgestaltung ist der weitere zweite Greifhebel auf einer Welle oder Achse an einer Strebe gelagert, die mit der Rückseite der Zahnstange verbunden ist, die von der Vorderseite mit der Zahnung abgewandt ist. Dies begünstigt eine raumsparende Ausführung. Gemäß einer bevorzugten Ausgestaltung wird der weitere zweite Greifhebel mittels eines Federelements in Richtung auf die zweite Aufnahme belastet. Gemäß einer bevorzugten Ausgestaltung ist das Federelement ein O-Ring, der den Aufnahmekörper umgibt und unter Vorspannung an der Außenseite des weiteren, zweiten Greifhebels anliegt.

Gemäß einer weiteren Ausgestaltung erstreckt sich die Zahnung über die gesamte Breite der Zahnstange und/oder weist die Klinke mindestens einen Zahn auf, der sich über weniger als die gesamte Breite der Zahnung erstreckt. Hierdurch wird ein seitlicher Austritt von Schmutz aus der Zahnung begünstigt. Gemäß einer weiteren Ausgestaltung hat jeder Klinkenzahn einen Klinkendurchbruch, der sich senkrecht zu einer Schwenkachse der Klinke durch den Klinkenzahn hindurch erstreckt. Der Klinkendurchbruch ist ein Kanal, der an der Zahnschneide und an den beiden Zahnflanken jedes Klinkenzahns geöffnet ist. Der Klinkendurchbruch vermeidet Schmutzansammlungen im Zentralbereich der Zahnung. Gemäß einer weiteren Ausgestaltung erstreckt sich der Klinkendurchbruch mittig durch jeden Klinkenzahn hindurch.

Gemäß einer weiteren Ausgestaltung stimmt der Abstand zwischen den beiden äußeren Enden jedes Klinkenzahns mit der Breite der Zahnung überein. Gemäß einer weiteren Ausgestaltung entspricht die Breite des Klinkendurchbruchs 1/4 bis 1/2, vorzugsweise etwa 1/3 des Abstands zwischen den beiden äußeren Enden des Klinkenzahns.

Gemäß einer weiteren Ausgestaltung ist das erste Mittel zum Verlagern ein teilweise aus dem Gehäuse herausragender Aufzugshebel, der mit einer an der Zahnstange und/oder dem Aufnahmekörper fixierten Auszugshebelhalterung verbunden ist. Der Aufzugshebel ist von außen betätigbar und ermöglicht ein einfaches Aufziehen von Flüssigkeit in eine Spritze. In dieser Hinsicht wird Bezug genommen auf die eingangs erwähnten Dokumente EP 0 656 229 B1 und US 5,620,660A, deren Inhalt hiermit in die vorliegende Anmeldung aufgenommen wird.

Gemäß einer weiteren Ausgestaltung weist die Zahnstange oben eine Ausnehmung für einen Nocken einer Resthubsperre auf. In dieser Hinsicht wird Bezug genommen auf die deutsche Patentanmeldung DE 10 2012 011 938.0 und die US-Provisional Nr. 61/661,018, deren Inhalt hiermit in die vorliegende Anmeldung aufgenommen wird.

Gemäß einer weiteren Ausgestaltung weist die Zahnstange einen C-förmigen und/oder einen U-förmigen Querschnitt auf, wobei die Zahnung an der Außenseite des Querbalkens des T-Querschnitts und/oder der Basis des U-Querschnitts angeordnet ist. Diese Ausgestaltung ist besonders stabil und vermeidet Einfallstellen und Materialanhäufungen des Kunststoffes. Bevorzugt hat die Zahnstange einen U-förmigen Querschnitt mit einem von der Innenseite der Basis vorstehenden vertikalen Balken, sodass sie zugleich einen T-Querschnitt aufweist.

Schließlich sind die Zahnstange und der Aufnahmekörper gemäß einer Ausgestaltung einteilig aus Kunststoff spritzgegossen und/oder aus PEEK oder aus einem anderen hochfesten Kunststoff hergestellt. Gemäß einer weiteren Ausgestaltung ist die Klinke aus Kunststoff spritzgegossen. Vorzugsweise ist die Klinke aus PEEK oder einem anderen hochfesten Kunststoff hergestellt.

Gemäß einer Ausgestaltung weist die Pipette einen Sensor zum Abtasten von Erhebungen und Vertiefungen auf dem Spritzenflansch der Spritzen auf, wie in der EP 0 657 216 B1 und der US 5,620,661A beschrieben, deren Inhalt hiermit in die vorliegende Anmeldung aufgenommen wird.

In der vorliegenden Anmeldung beziehen sich die Angaben "oben" und "unten", "vorn" und "hinten" sowie davon abgeleitete Begriffe auf die Ausrichtung der Pipette, bei der das stangenförmige Gehäuse vertikal ausgerichtet ist und die Aufnahme für die Spritze unten angeordnet ist und die Zahnung dem Betrachter zugewandt ist.

Die Erfindung wird nachfolgend anhand der anliegenden Zeichnungen eines Ausführungsbeispiels näher erläutert. In den Zeichnungen zeigen:
- Fig. 1: eine erfindungsgemäßer Dispenser mit darin gehaltener Spritze in einer Perspektivansicht von der Seite;
- Fig. 2: denselben Dispenser in einem grobschematischen Längsschnitt;
- Fig. 3: Zahnstange mit Aufnahmekörper desselben Dispensers in einer Seitenansicht;
- Fig. 4: Zahnstange und Aufnahmekörper desselben Dispensers in einer Vorderansicht;
- Fig. 5: Aufnahmekörper desselben Dispensers in einer vergrößerten Unteransicht;
- Fig. 6: Zahnstange und Aufnahmekörper desselben Dispensers in einer Perspektivansicht schräg von vorn und von der Seite;
- Fig. 7: Zahnstange und Aufnahmekörper desselben Dispensers in einer Perspektivansicht schräg von vorn und von der anderen Seite;
- Fig. 8: Zahnstange und Aufnahmekörper mit Schieber desselben Dispensers in einer Perspektivansicht schräg von vorn und von der Seite;
- Fig. 9: Zahnstange und Aufnahmekörper mit Schieber desselben Dispensers in einer Perspektivansicht schräg von vorn und von der anderen Seite;
- Fig. 10: Klinke desselben Dispensers in einer vergrößerten Perspektivansicht schräg von hinten und von der Seite;
- Fig. 11: denselben Dispenser bei abgenommener vorderer Gehäusehälfte in einer Perspektivansicht schräg von vorn und von der Seite;
- Fig. 12: ein oberes Rahmenteil mit darin angeordneten Bauteilen derselben Pipette bei abgenommenem Gehäuse in einer vergrößerten Perspektivansicht;
- Fig. 13: dieselbe Anordnung in einem Längsschnitt;
- Fig. 14: Zahnstange mit Betätigungselement, Klinke und Abdeckung derselben Pipette in einer weiter vergrößerten Perspektivansicht;
- Fig. 15: Abdeckung derselben Pipette in einer weiteren Perspektivansicht;
- Fig. 16: vergrößertes Detail derselben Abdeckung bei entnommenem Exzenterteil in einer weiteren Perspektivansicht;
- Fig. 17: die Abdeckung in einer weiteren Perspektivansicht;
- Fig. 18: die Abdeckung in einem perspektivischen Sprengbild;
- Fig. 19: das Exzenterteil in einer weiter vergrößerten Perspektivansicht;
- Fig. 20: das Exzenterteil in einer Ansicht von einer gegenüberliegenden Seite;
- Fig. 21: die Abdeckung in einer Justierstellung mit der Einfallkante in der untersten Stellung in Vorderansicht;
- Fig. 22: die Abdeckung in einer Justierstellung mit der Einfallkante in der mittleren Stellung in Vorderansicht;
- Fig. 23: die Abdeckung in einer Justierstellung mit der Einfallkante in der obersten Stellung in einer Seitenansicht;
- Fig. 24: das Wahlrad in einer Ansicht von der Unterseite;
- Fig. 25: die Pipette beim Betätigen des Betätigungselements vor dem Eingriff der Klinke in die Zahnung in einem grobschematischen Längsschnitt;
- Fig. 26: die Pipette in derselben Situation in einer vergrößerten Detailansicht;
- Fig. 27: die Pipette beim optimalen Eingriff der Klinke in die Zahnung in einem grobschematischen Längsschnitt;
- Fig. 28: die Pipette in derselben Situation in einer vergrößerten Detailansicht;
- Fig. 29: die Pipette bei einem ungünstigen Eingriff der Klinke in die Zahnung in einem grobschematischen Längsschnitt;
- Fig. 30: die Pipette in derselben Situation in einer vergrößerten Detailansicht.

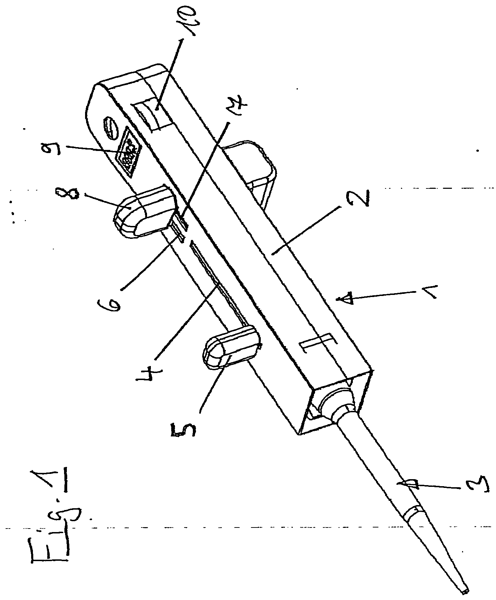

Gemäß Fig. 1 hat eine Pipette 1 ein stangenförmiges Gehäuse 2, in dem unten eine Spritze 3 gehalten ist. Von einer Seitenwand des Gehäuses 2 steht über einem gradlinigen Schlitz 4 ein Aufzugshebel 5 von dem Gehäuse 2 vor. Von derselben Seitenwand des Gehäuses 2 steht über zwei weiteren Schlitzen 6, 7 ein Bedienknopf 8 einer Zahnstangen-Klinkensteuerung vor. Darüber ist in dieselbe Seitenwand des Gehäuses 2 eine Anzeigeeinrichtung in Form eines Displays 9 eingelassen. Aus Öffnungen in der benachbarten Seitenwand stehen Segmente eines Wahlrades 10 heraus.

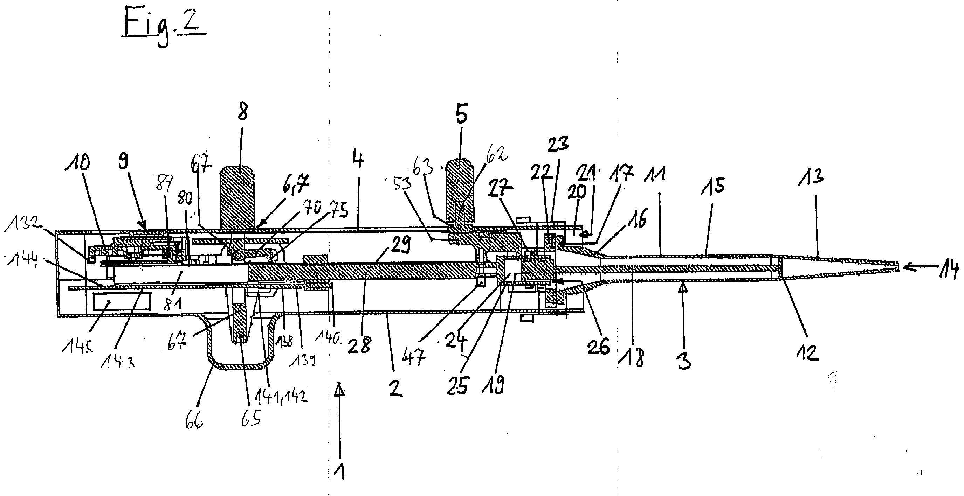

Gemäß Fig. 2 weist die Spritze 3 einen Zylinder 11 und einen darin verschieblich angeordneten Kolben 12 auf. Der Zylinder 11 hat unten einen konischen Abschnitt 13 mit einem Loch 14 für den Durchgang von Flüssigkeiten und darüber einen zylindrischen Abschnitt 15, in dem der Kolben 12 verlagerbar ist. Oben hat der Zylinder 11 einen ersten Befestigungsabschnitt 16 mit einem umlaufenden Flansch 17. Vom Kolben 12 steht nach oben eine Kolbenstange 18 vor, die einen zweiten Befestigungsabschnitt 19 mit mehreren umlaufenden Wulsten aufweist.

Die Spritze 3 ist mit dem Flansch 17 in einer ersten Aufnahme 20 am unteren Ende des Gehäuses 2 angeordnet, die am unteren Ende des Gehäuses 2 eine axial gerichtete erste Öffnung 21 zum Einsetzen und Entnehmen der Spritze 3 aufweist. Die Spritze 3 drückt mit der Oberseite gegen einen druckempfindlichen Ringsensor 22, der Vorsprünge am oberen Rand des Flansches 17 abtastet. Der auf dem Flansch 17 angegebene Code bezeichnet die Größe der jeweiligen Spritze 3. Der Flansch 17 ist in dieser Stellung mittels ersten Mitteln zum lösbaren Halten in Form von ersten Greifhebeln 23 im Gehäuse 2 gehalten.

Der zweite Befestigungsabschnitt 19 des Kolbens 12 ist in einer zweiten Aufnahme 24 in einem hohlzylindrischen Aufnahmekörper 25 angeordnet. Dieser weist zum Einsetzen des zweiten Befestigungsabschnittes 19 eine axial gerichtete zweite Öffnung 26 auf. Der zweite Befestigungsabschnitt 19 ist mittels zweiter Mittel zum lösbaren Halten in Form von zweiten Greifhebeln 27 gehalten, die zwischen die Wulste des zweiten Befestigungsabschnittes 19 eingreifen oder diesen einklemmen.

Der Aufnahmekörper 25 ist fest mit einer Zahnstange 28 mit einer Zahnung 29 verbunden, die sich unterhalb des Schlitzes 4 in Längsrichtung des Gehäuses 2 erstreckt.

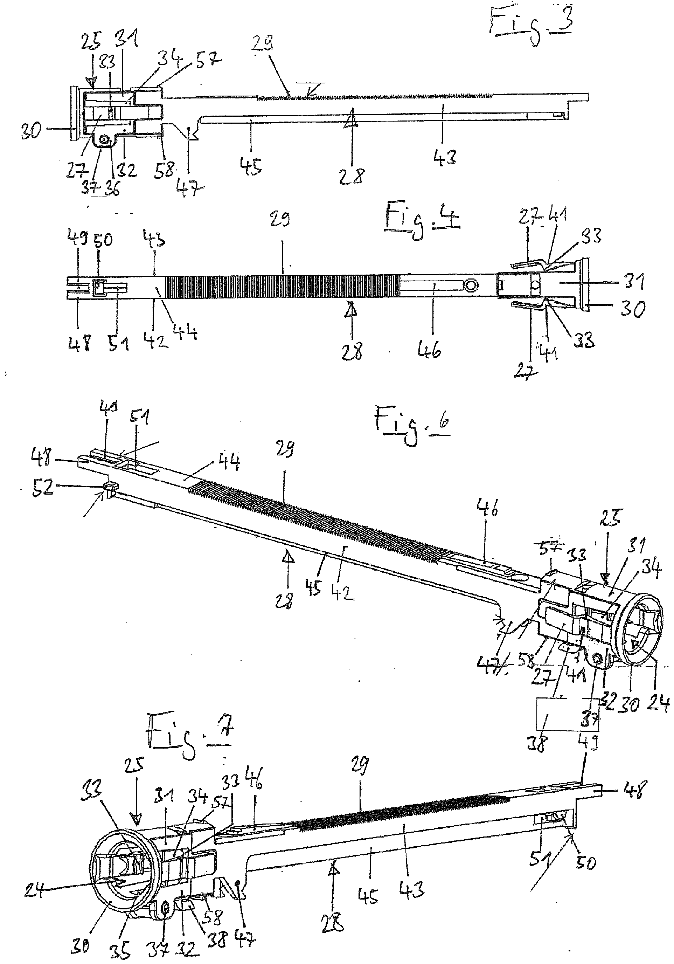

Gemäß Fig. 3-7 sind die Zahnstange 28 und der Aufnahmekörper 25 einteilig aus Kunststoff hergestellt. Der Aufnahmekörper 25 ist im Wesentlichen topfförmig und an seinem Boden einteilig mit dem unteren Ende der Zahnstange 28 verbunden. Der Aufnahmekörper 25 ist als Käfig ausgestaltet, der am unteren Ende ein Ringelement 30 und von dem Ringelement ausgehend parallel nach oben erstreckte Streben 31, 32 aufweist. Die Streben 31,32 sind an ihrem oberen Ende mit dem unteren Ende der Zahnstange 28 verbunden. Die vordere Strebe 31 ist mit der Vorderseite der Zahnstange 28 verbunden, welche die Zahnung 29 aufweist. Die hintere Strebe 32 ist mit der von der Zahnung 29 abgewandten Rückseite der Zahnstange 28 verbunden.

Das Ringelement 30 umgrenzt die zweite Öffnung 26 des Aufnahmekörpers 25. An der zweiten Öffnung 26 ist der Aufnahmekörper 25 trichterförmig nach unten erweitert.

Die beiden zweiten Greifhebel 27 sind einarmig. An ihrem unteren Ende sind sie mit dem Ringelement 30 verbunden und erstrecken sich im Wesentlichen parallel zu den Streben 31, 32 nach oben. Die zweiten Greifhebel 27 weisen in einem Abstand von ihren Verbindungen mit dem Ringelement 30 eine Abwinklung nach innen auf, die innen vorstehende Rastvorsprünge 33 bildet. Die zweiten Greifhebel 27 sind neben seitlichen Öffnungen 34 des Aufnahmekörpers 25 zwischen den beiden Streben 31,32 angeordnet, sodass sie elastisch in die seitlichen Öffnungen hineinbiegbar sind.

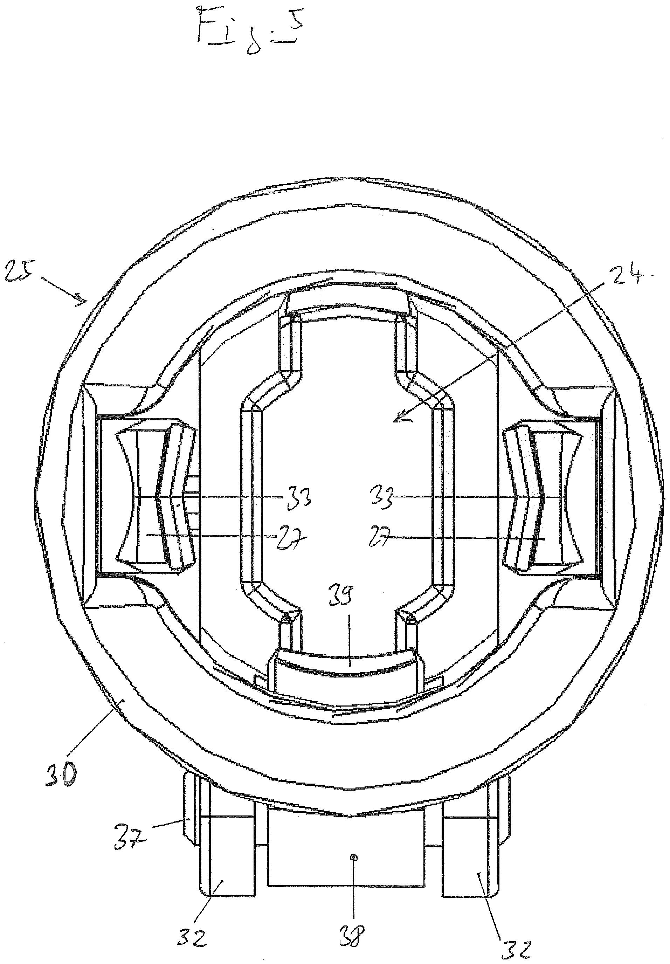

Die hintere Strebe 32 weist eine weitere seitliche Öffnung in Form eines rechteckigen Langloches 35 auf (vgl. Fig. 7). An den beiden Längsseiten des Langlochs 35 stehen Lageraugen 36 nach außen vor. In den Lageraugen 36 ist auf einem Stift 37 ein weiterer zweiter Greifhebel 38 gelagert, der ebenfalls einarmig ist. Der weitere zweite Greifhebel 38 hat in einem Abstand von dem Stift 37 eine Abwinklung nach innen, die einen nach innen vorstehenden weiteren Rastvorsprung 39 bildet (vgl. Fig. 5). Der weitere zweite Greifhebel 38 ist in das Langloch 35 hinein in die zweite Aufnahme 24 im Aufnahmekörper 25 schwenkbar.

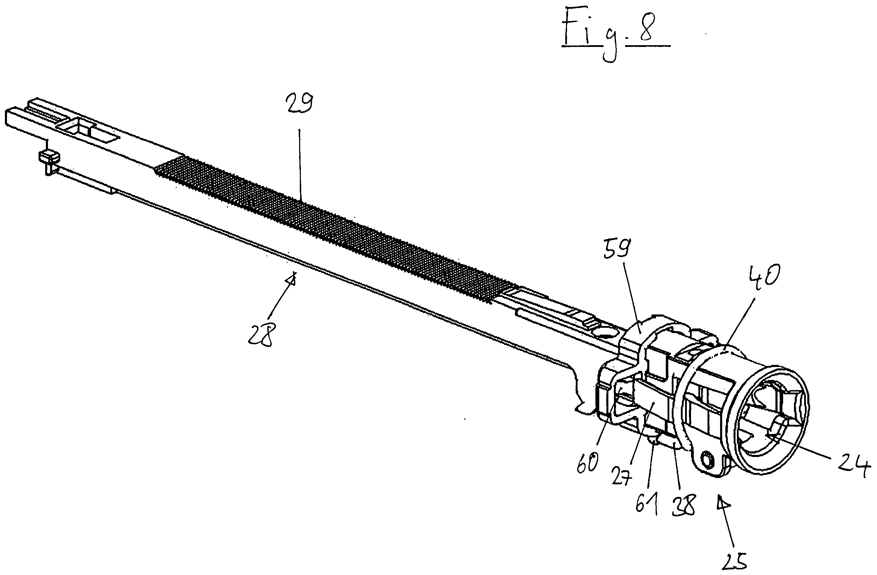

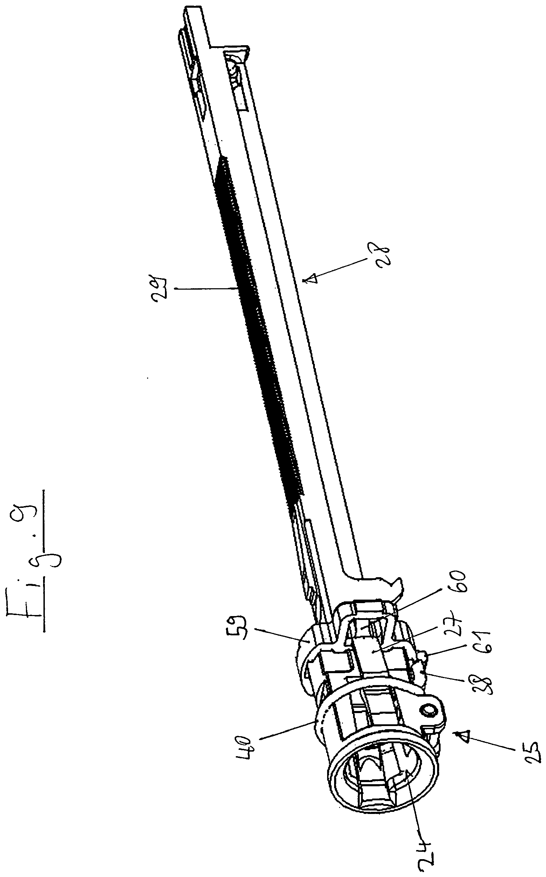

Gemäß Fig. 8 und 9 sitzt ein O-Ring 40 unter Vorspannung außen auf dem Aufnahmekörper 25. Der O-Ring 40 ist in Vertiefungen 41 außen an den Abwinklungen der zweiten Greifhebel 27 geführt. Der O-Ring 40 belastet die zweiten Greifhebel 27, 38, sodass sie in die zweite Aufnahme 24 des Aufnahmekörpers 25 hineingeschwenkt werden.

Die Zahnstange 28 hat einen U-förmigen Querschnitt mit einem zwischen den beiden seitlichen Schenkeln 42, 43 von der Innenseite der Basis 44 vorstehenden mittleren Schenkel 45, sodass die Zahnstange 28 zugleich einen T-Querschnitt aufweist. Der mittlere Schenkel 45 steht weiter von der Basis 44 vor als die beiden seitlichen Schenkel 42, 43. Der mittlere Schenkel 45 dient der Führung der Zahnstange 28 in einem Lagerbock.

Die Zahnung 29 erstreckt sich über einen Teil des Abstandes zwischen dem unteren und dem oberen Ende der Zahnstange 28. Ausgehend vom unteren Ende der Zahnung 29 erstreckt sich auf der Vorderseite der Zahnstange 28 eine längsgerichtete Rippe 46, deren Höhe nach unten zunimmt.

Auf der von der Zahnung 29 abgewandten Rückseite der Zahnstange 28 steht ein unterer Anschlag 47 nach außen vor. Der untere Anschlag hat eine hakenförmige Kontur an der Oberseite.

Am oberen Ende hat die Zahnstange 28 eine nach oben vorstehende Zunge 48, an der ein Längsschlitz 49 vorhanden ist, der sich in Längsrichtung der Zahnstange 28 erstreckt. Unter dem Längsschlitz 49 hat die Zahnstange 28 eine axiale Bohrung 50. Darunter weist sie eine kastenförmige Aufnahme 51 für eine Mutter und das darin eingeschraubte Ende eine Einstellschraube auf.

Neben der Aufnahme steht von einer Seite der Zahnstange 28 eine Führungsrippe 52 vor.

Die Zahnstange 28 und der Aufnahmekörper 25 sind einteilig aus PEEK spritzgegossen.

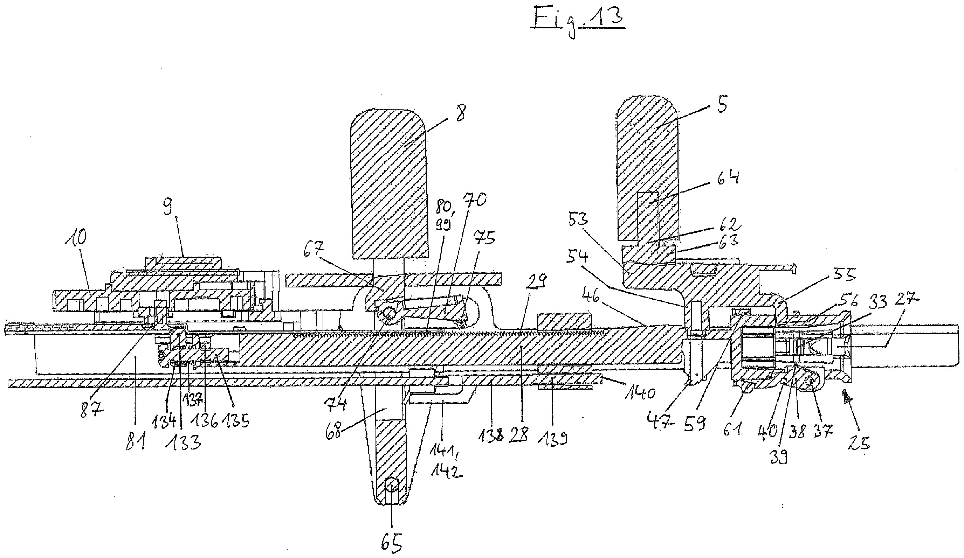

Gemäß Fig. 2 und 13 ist eine Aufzugshebelhalterung 53 an dem Aufnahmekörper 25 und an einem unteren Teil der Zahnstange 28 fixiert. Die Aufzugshebelhalterung 53 ist an einem Sockel 54 unterhalb der Rippe 46 mittels einer Schraube an der Zahnstange 28 fixiert. Die Schraube ist durch ein Loch von der Rückseite der Zahnstange 28 aus in eine Gewindebohrung im Sockel 54 eingeschraubt. Zudem stützt sich die Aufzugshebelhalterung 53 über einen Arm 55 in einer Vertiefung 56 an der Außenseite der vorderen Strebe 31 ab.

Von den oberen Enden der Streben 31, 32 stehen kleine Rastelemente 57, 58 nach außen vor (vgl. Fig. 6). Über die Rastelemente 57, 58 ist von oben ein Schieber 59 auf das obere Ende des Aufnahmekörpers 25 aufgeschoben (vgl. Fig. 8 und 9). Der Schieber 59 weist an zwei einander gegenüberliegenden Seiten Rampen 60 und hinten eine Rampe 61 auf. Der Abstand der Rampen 60, 61 von der Zahnstange 28 nimmt nach oben zu. Der Schieber 59 ist nach unten verlagerbar, sodass die Rampen 60, 61 die oberen Enden der zweiten Greifhebel 27, 38 innen kontaktieren und bei weiterer Verlagerung des Schiebers 59 nach unten die zweiten Greifhebel 27, 38 auseinanderspreizen.

Gemäß Fig. 2 und 13 ist ein Aufzugshebelträger 62 vorhanden, der mit einer Gleitplatte 63 an den Rändern des Schlitzes 4 anliegt. Der Aufzugshebelträger 62 weist einen nach oben vorstehenden Pfosten 64 auf, der den Schlitz 4 durchgreift. Auf dem Pfosten 64 ist außerhalb des Gehäuses 2 der Aufzugshebel 5 fixiert.

Am Ende der Verlagerung des Aufnahmekörpers 25 zur ersten Aufnahme 20 hin trifft die Gleitplatte 63 auf den unteren Rand des Schlitzes 4, wodurch ein unterer Anschlag gegeben ist.

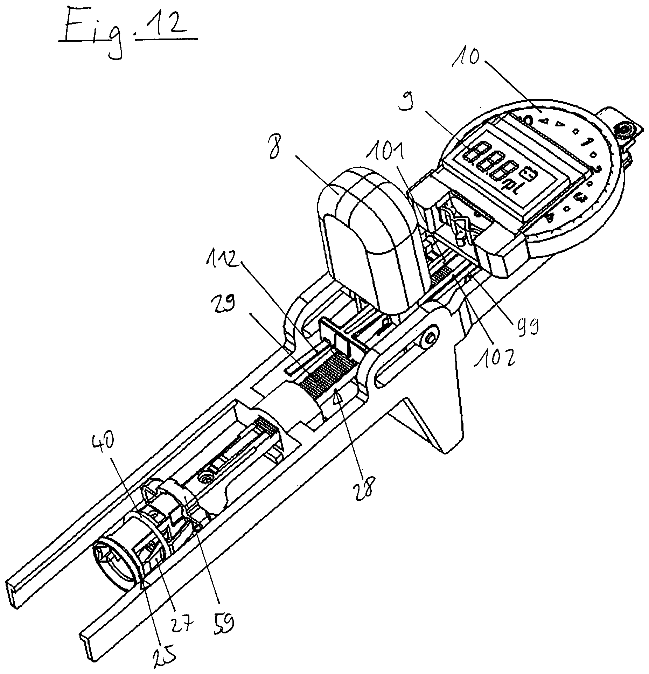

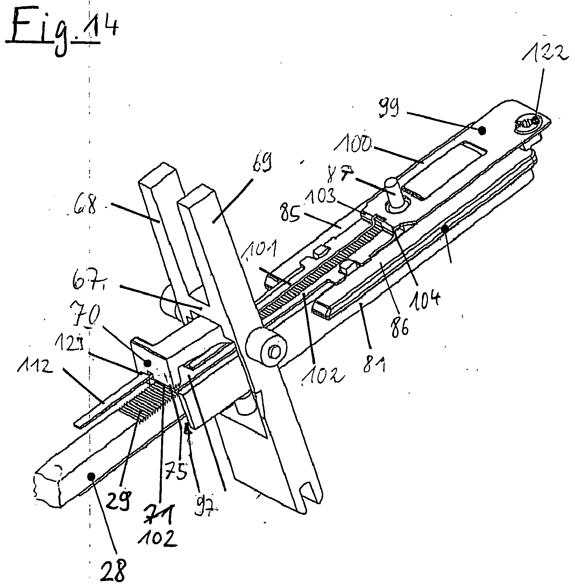

In der oberen Hälfte des Gehäuses 2 ist in einem Schwenklager 65 in einer Ausbuchtung 66 der dem Schlitz 4 gegenüberliegenden Seitenwand des Gehäuses 2 ein Betätigungselement in Form eines Dosierhebels 67 schwenkbar gelagert. Der Dosierhebel 67 hat gemäß Fig. 12 und 14 zwei voneinander beabstandete Schenkel 68, 69, die auf der gegenüberliegenden Seitenwand des Gehäuses 2 aus den beiden Schlitzen 6, 7 herausragen. Dort ist der Bedienknopf 8 auf den herausragenden Enden der Schenkel 68, 69 fixiert.

Am Ende der Verlagerung des Aufnahmekörpers 25 von der ersten Aufnahme 20 weg nach oben trifft der untere Anschlag 47 auf einen fest im Gehäuse 2 angeordneten Lagerkörper, in dem das Schwenklager 65 für den Dosierhebel 67 ausgebildet ist. Hierdurch ist der obere Anschlag für die Verlagerung des Aufnahmekörpers 25 gegeben.

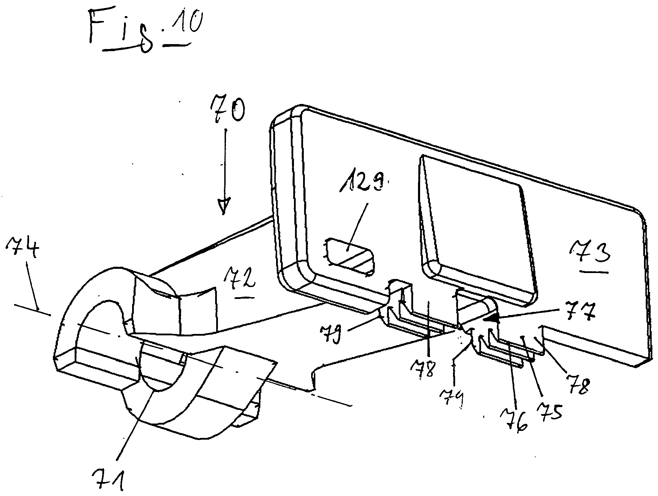

Zwischen den beiden Schenkeln 68, 69 des Dosierhebels 67 ist eine Klinke 70 an einem Gleitlager 71 schwenkbar gelagert. Gemäß Fig. 10 hat die Klinke 70 einen Klinkenarm 72, der einenends mit dem Gleitlager 71 und anderenends mit einem Zahnträger 73 verbunden ist. Der Zahnträger 73 ist eine senkrecht zum Klinkenarm 72 ausgerichtete Platte. An einem zur Schwenkachse 74 des Gleitlagers 71 parallelen Rand trägt der Zahnträger 73 mehrere parallele (im Beispiel 3) senkrecht zum Klinkenarm 72 erstreckte Klinkenzähne 75 mit Zahnschneiden 76 an den freien Enden. Diese sind in der Mitte durch einen Klinkendurchbruch 77 unterbrochen, der sich senkrecht zur Schwenkachse 74 erstreckt. Der Klinkendurchbruch 77 hat die Form eines Kanals, der auf der Seite der Zahnschneiden 76 geöffnet ist. Zudem ist der Klinkendurchbruch zu den äußeren Zahnflanken 78, 79 der Klinkenzähne hin geöffnet.

Die Klinke 70 ist mit den Klinkenzähnen 75 oberhalb der Zahnung 29 der Zahnstange 28 angeordnet (vgl. Fig. 2, 12, 13).

Der Dosierhebel 67 wird von einer Federeinrichtung in die Stellung von Fig. 2 gedrückt. Entgegen der Wirkung der Federeinrichtung ist der Dosierhebel 67 durch Betätigen des Bedienknopfes 8 nach unten schwenkbar. Hierbei wird die Klinke 70 mittels einer weiteren Federeinrichtung zu der Zahnstange 28 hingedrückt, bis sie mit den Klinkenzähnen 75 in die Zahnung 29 einfällt und die Zahnstange 28 nach unten vorschiebt.

Die Klinke 70 ist vorzugsweise einteilig aus Kunststoff oder Metall hergestellt. Die Klinke 70 ist vorzugsweise aus PEEK hergestellt. Vorzugsweise ist sie spritzgegossen.

Zwischen der Klinke 70 und der Zahnstange 28 ist eine verschiebbare Abdeckung 80 angeordnet. Die Abdeckung 80 ist durch Drehen des seitlich aus dem Gehäuse 2 herausstehenden Wahlrades 10 verlagerbar, sodass sie die Zahnung 29 der Zahnstange 28 mehr oder weniger überdeckt. Die Klinke 70 wird beim Betätigen des Bedienknopfes 8 zunächst gegen die Abdeckung 80 gedrückt und fällt darunter in die Zahnung 29 ein. Deshalb hängt das Ausmaß der Verlagerung der Zahnstange 28 beim Schwenken des Dosierhebels 67 nach unten von der Lage der Abdeckung 80 bezüglich der Zahnung 29 ab.

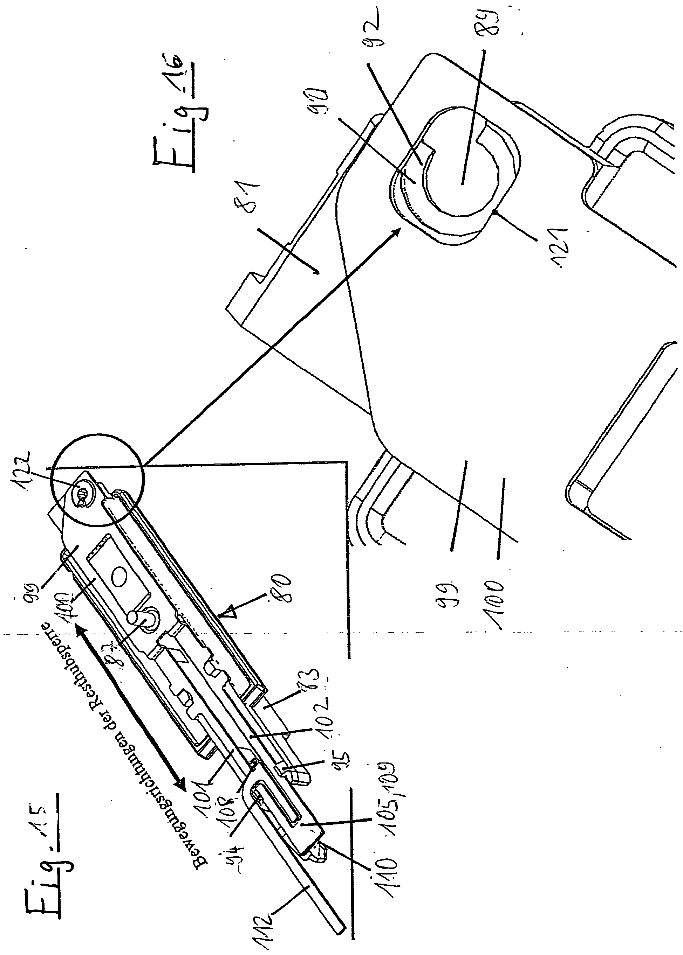

Die Abdeckung 80 weist gemäß Fig. 15 bis 18 ein erstes Abdeckungsteil 81 in Form eines langgestreckten Hohlkörpers auf. Das erste Abdeckungsteil 81 weist zwei parallele, streifenförmige Seitenteile 82, 83 auf. Diese sind an den vorderen Rändern im oberen Bereich durch ein plattenförmiges Überbrückungsteil 84 überbrückt. Senkrecht von den Außenseiten der Seitenteile 82, 83 stehen streifenförmige Flügelteile 85, 86 vor.

Nahe dem unteren Ende des Überbrückungsteiles 84 steht von diesem senkrecht ein Kopplungselement in Form eines Stiftes 87 vor. Zudem ist auf der Vorderseite des Überbrückungsteiles 84 ein plattenförmiger Nutenstein 88 vorhanden.

Schließlich weist das Überbrückungsteil 84 in der Nähe des oberen Endes eine Lagerbohrung 89 auf, die sich senkrecht durch das Überbrückungsteil 84 hindurch erstreckt. Die Lagerbohrung 89 ist in einem Abschnitt des Überbrückungsteils 84 ausgebildet, der über das Seitenteil 83 hinaussteht. Die Lagerbohrung 89 weist an der Vorderseite eine vordere Vertiefung 90 und an der Unterseite eine hintere Vertiefung 91 auf. Dazwischen läuft in der Lagerbohrung 89 ein kreisringscheibenförmiger Bohrungsrand 92 um.

Das Überbrückungsteil 84 weist an einem oberen Ende eine erste Aussparung 93 auf und die Lagerbohrung 89 ist zu der Aussparung 93 hin geöffnet.

Vom vorderen Rand der Seitenteile 82, 83 stehen einander zugewandte Führungshaken 94, 95 vor.

Auf der hinteren Seite des Überbrückungsteiles 84 ist eine Längsnut 96 vorhanden, die sich in Längsrichtung des ersten Abdeckungsteils erstreckt und zu einem Kanal 97 im Abstandsbereich zwischen den Seitenteilen 82, 83 hin geöffnet ist. Unterhalb des Überbrückungsteils 84 ist zwischen den vorderen Rändern der Seitenteile 82, 83 ein weiterer Längsschlitz 98 vorhanden.

Das erste Abdeckungsteil 81 ist vorzugsweise einteilig aus Kunststoff spritzgegossen.

Ferner weist die Abdeckung 80 ein zweites Abdeckungsteil 99 auf, das im Wesentlichen streifenförmig ist. Das zweite Abdeckungsteil 99 hat oben einen plattenförmigen Kopfabschnitt 100 und unten zwei parallele streifenförmige Mittelabschnitte 101, 102, die über gegenläufige Abwinklungen 103, 104 mit dem Kopfabschnitt 100 verbunden sind.

Der Mittelabschnitt 102 hat am unteren Ende einen U-förmigen Fußabschnitt 105. Der Fußabschnitt 105 ist an einem Schenkel mit dem Mittelabschnitt 102 verbunden und der andere Schenkel ist auf den Mittelabschnitt 101 ausgerichtet. Der Mittelabschnitt 101 ist am unteren Ende über einen ersten Verbindungsabschnitt 106 mit einem mittleren Streifenabschnitt 107 verbunden, der am oberen Ende eine vorstehende Abwinklung 108 aufweist. Der mittlere Streifenabschnitt 107 ist zwischen den Schenkeln des U-förmigen Fußabschnittes 105 angeordnet.

Auf der vorderen Seite des Fußabschnittes 105 ist eine Abhaltefläche 109 vorhanden. Der untere Rand des Fußabschnittes 105 ist eine Einfallkante 110. Die Klinke 70 wird über die Abhaltefläche 109 verlagert und fällt nach dem Passieren der Einfallkante 110 in die Zahnung 29 ein.

Ferner ist der Mittelabschnitt 101 am unteren Ende über einen zweiten Verbindungsabschnitt 111 mit einer Federzunge 112 verbunden, die neben dem Fußabschnitt 105 angeordnet ist und sich parallel zu den Mittelabschnitten 101, 102 nach unten erstreckt.

Der Kopfabschnitt 100 weist ein großes Loch 113 auf, das den Stift 87 mit Spiel aufnimmt. Ferner weist der Kopfabschnitt 100 eine große rechteckige Ausstanzung 114 auf, die den Nutenstein 88 mit axialem Spiel aufnimmt.

Die beiden Mittelabschnitte 101, 102 weisen an den voneinander abgewandten Rändern zwischen vorstehenden Zungen 115 bis 118 Freiräume 119, 120 auf. Die Führungshaken 94, 95 greifen mit axialem Spiel in die Freiräume 119, 120 ein und übergreifen die Mittelabschnitte 101, 102 randseitig.

Somit ist der zweite Abdeckungsteil 99 am ersten Abdeckungsteil 81 gehalten, in Längsrichtung am ersten Abdeckungsteil 81 geführt und in Längsrichtung bezüglich des ersten Abdeckungsteils 81 verlagerbar.

Der Kopfabschnitt 100 weist zudem an einer oberen Ecke eine Exzenteraufnahme 121 auf. Die Exzenteraufnahme 121 ist annähernd rechteckig, wobei die Ecken ausgerundet sind. Die Exzenteraufnahme 121 ist oberhalb der Lagerbohrung 89 angeordnet, wobei die kürzere Seite der Exzenteraufnahme 121 in Längsrichtung des ersten Abdeckungsteils 81 angeordnet ist und die längere Seite senkrecht dazu.

Das zweite Abdeckungsteil 99 ist beispielsweise einteilig aus einem Blech aus Federstahl durch Stanzen und Biegen hergestellt.

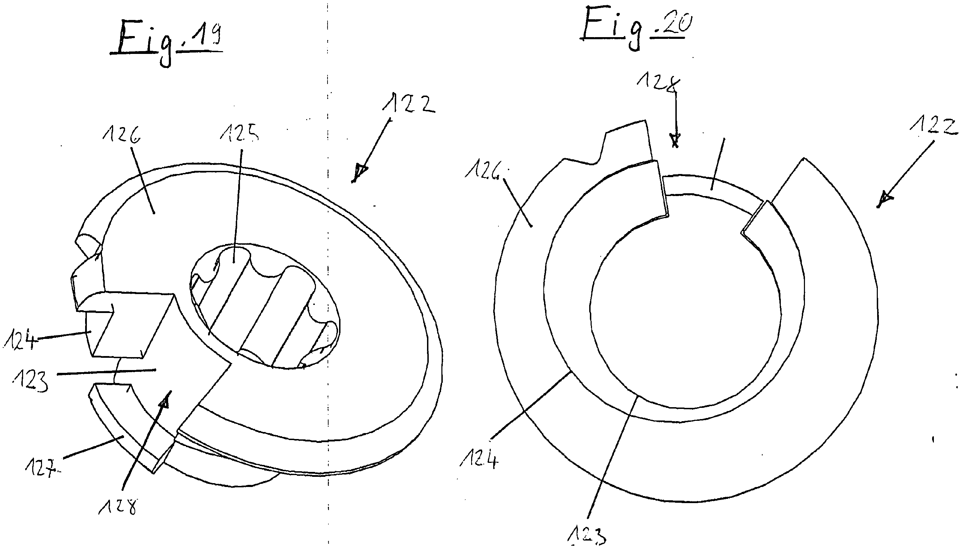

Ferner umfasst die Abdeckung 80 ein Exzenterteil 122. Das Exzenterteil 122 hat gemäß Fig. 19 und 20 eine Welle 123, auf der ein Exzenter 124 angeordnet ist. Der Durchmesser des Exzenters 124 entspricht der Länge der kürzeren Seite der Exzenteraufnahme 121. An einem Ende der Welle 123 ist ein Werkzeugangriff 125 (z.B. Torx, Inbus, Kreuzschlitz oder Schlitz) vorhanden, der von einer Stirnseite der Welle 123 aus zugänglich ist. An diesem Ende ist auf der Welle 123 eine radial vorstehende Scheibe 126 angeordnet. Am anderen Ende hat die Welle 123 einen radial vorstehenden Rastvorsprung 127 in Form eines Kreisscheibensegments.

Die Scheibe 126 und der Exzenter 124 weisen aus fertigungstechnischen Gründen eine zweite Aussparung 128 auf, deren radiale Begrenzungen mit dem Rastvorsprung 127 fluchten.

Das Exzenterteil 122 ist in axialer Richtung der Lagerbohrung in die Exzenteraufnahme 121 und die Lagerbohrung 89 eingesetzt, sodass die Scheibe 126 das zweite Abdeckungsteil 99 übergreift. Der Rastvorsprung 127 ist durch die erste Aussparung 93 an der Ecke des Überbrückungsteils 84 hindurch eingeführt und unter den Bohrungsrand 92 gedreht. Der Exzenter 124 ist im Bereich der Exzenteraufnahme 121 und der vorderen Vertiefung 90 angeordnet. Der Rastvorsprung 127 untergreift den Bohrungsrand 92 und greift in die hintere Vertiefung 91 ein.

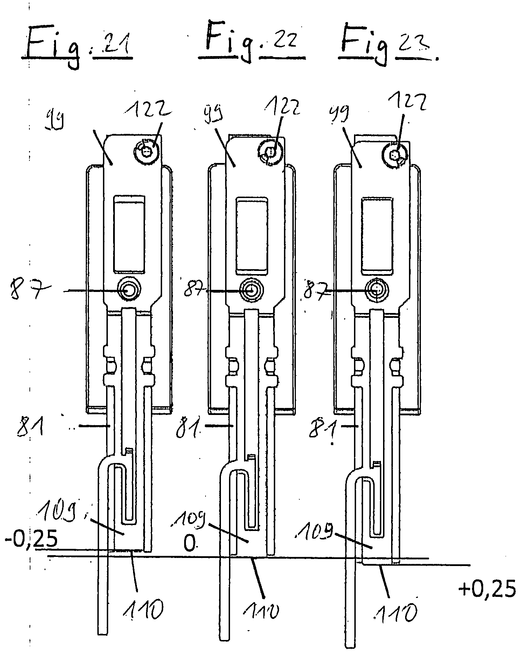

Das Exzenterteil 122 ist durch Ansetzen eines Werkzeuges an den Werkzeugangriff 125 drehbar. Hierbei verlagert der Exzenter 124 das zweite Abdeckungsteil 99bezüglich des ersten Abdeckungsteils 81 in Axialrichtung. In Fig. 21 und 23 sind die beiden Extrempositionen des zweiten Abdeckungsteiles 99 bezüglich des ersten Abdeckungsteiles 81 bei dieser Verlagerung gezeigt, die von der mittleren Lage in Fig. 22 um +/- 0,25 mm entfernt sind.

Gemäß Fig. 2 und 14 erstreckt sich die Zahnstange 28 durch den Kanal 97 des ersten Abdeckteiles 81 hindurch. Die Klinke 70 ist mit einem Führungsschlitz 129 am unteren Ende auf die Federzunge 112 des zweiten Abdeckteiles 99 aufgefädelt. An den Flügelteilen 85, 86 ist die Abdeckung 80 in Längsrichtung verschiebbar im Gehäuse geführt.

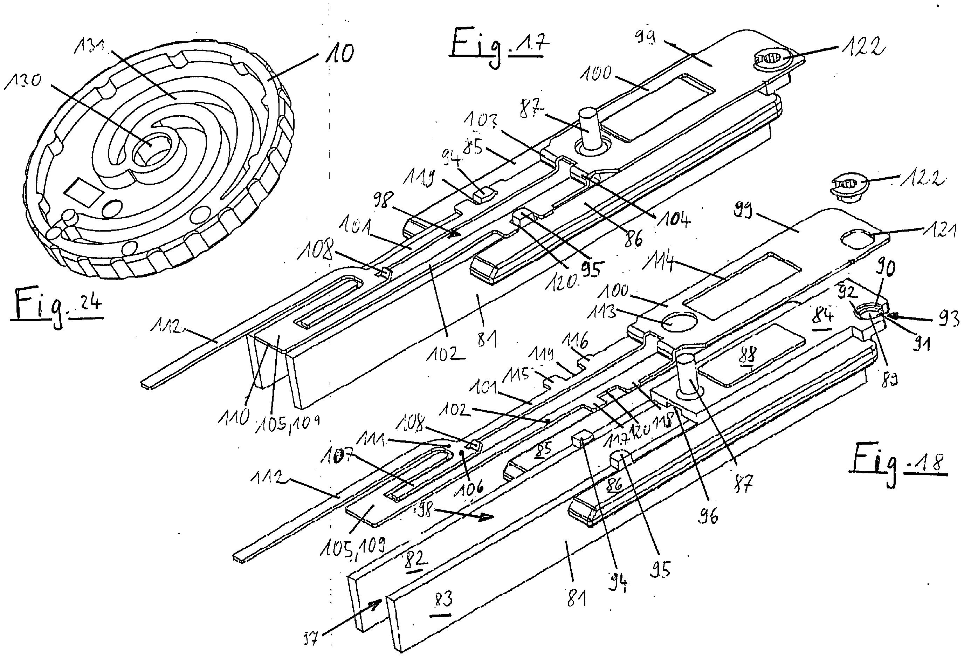

Durch Drehen des Wahlrads 10 ist die Abdeckung 80 in Längsrichtung verlagerbar. Hierfür hat das Wahlrad 10 gemäß Fig. 24 ein zentrales Lagerloch 130 und eine spiralförmige Führungskurve 131. Das Wahlrad 10 ist an dem Lagerloch 130 drehbar auf einer Achse am Gehäuse 2 gelagert und der Stift 87 der Abdeckung 80 greift in die Führungskurve 131 ein. Infolgedessen wird bei einem Drehen des Wahlrades 10 die Abdeckung 80 entlang ihrer Führung im Gehäuse 2 verlagert.

Dem Wahlrad 10 ist ein weiterer Sensor 132 zugeordnet, der die Drehstellung des Wahlrades 10 erfasst.

Gemäß Fig. 13 ist am oberen Ende der Zahnstange 28 ein Nocken 133 angeordnet. Der Nocken 133 steht aus dem Längsschlitz 49 der Zahnstange 28 nach außen vor. Unter dem weiteren Längsschlitz 49 weist der Nocken 133 eine axiale Bohrung 134 auf. Eine Einstellschraube 135 ist durch die axiale Bohrung 134 hindurch in eine Mutter 136 am oberen Ende der Zahnstange 28 eingeschraubt. Eine Schraubenfeder 137 ist auf der Einstellschraube 135 geführt und stützt sich einenends am Schraubenkopf der Einstellschraube 135 und anderenends am oberen Ende der Zahnstange 28 ab, um den Nocken 133 in der Anlage am Schraubenkopf zu halten.

Bei einer Verlagerung der Zahnstange 28 nach unten wird der Nocken 133 durch die Längsnut 96 des Überbrückungsteils 84 hindurchbewegt und tritt zwischen den gegenläufigen Abwinklungen 103, 104 aus dem weiteren Längsschlitz 98 der Abdeckung 80 aus. Schließlich trifft der Nocken 133 auf die vorstehende Abwinklung 108 und drückt diese gemeinsam mit der Federzunge 112 nach vorn. Hierdurch wird die auf der Federzunge 112 geführte Klinke 70 aus der Zahnung 29 ausgehoben und eine Resthubsperre verwirklicht.

Neben der Zahnstange 28 ist ein Übertragungsglied 138 zum Steuern eines Umkehrhubes im Gehäuse der Pipette angeordnet (vgl. Fig. 13). Das Übertragungsglied 138 hat einen streifenförmigen Übertragungsabschnitt 139, der parallel zur Zahnstange 28 im Gehäuse 2 der Pipette geführt und verlagerbar ist.

Der streifenförmige Übertragungsabschnitt 139 hat ein unteres Übertragungsgliedende 140, das sich bei einer Verlagerung des Übertragungsgliedes 138 nach unten im Verlagerungsbereich eines von der Seite der Zahnstange 28 vorstehenden unteren Anschlages befindet.

Mit dem oberen Ende des streifenförmigen Übertragungsabschnittes 139 sind zwei Steuerungsarme 141, 142 verbunden, die parallel zueinander in einer Ebene senkrecht zur Zeichenebene angeordnet sind.

Das Übertragungsglied 138 ist so verlagerbar, dass die Steuerungsarme 141, 142 mit ihren oberen Enden in den Verlagerungsbereich von angeschrägten Anschlagkanten an den Schenkeln 68, 69 gelangen.

Das Übertragungsglied 138 und die damit zusammenwirkenden Bauteile sind vorzugsweise so ausgebildet, wie in der europäischen Patentanmeldung 120 02 849.3-2113 und in der U.S.-Provisionalanmeldung Nr. 61/636,977 beschrieben, deren Inhalt hiermit in die vorliegende Anmeldung aufgenommen wird.

Ferner ist in der oberen Hälfte des Gehäuses 2 eine Leiterplatte 143 mit einer Elektronik 144 angeordnet (vgl. Fig. 2). Dort befinden sich auch eine elektrische Spannungsversorgung in Form von Batterien oder Akkus 145. Die Elektronik 144 ist mit dem Ringsensor 22, dem weiteren Sensor 132 zum Erfassen der Drehstellung des Wahlrades 10 und dem Display 9 verdrahtet. Ferner ist die Elektronik 144 an den Akku 145 angeschlossen.

Die Elektronik 144 ermittelt aus den vom Ringsensor 22 gelieferten Messsignalen die jeweilige Spritzengröße und aus der Einstellung des Wahlrades 10 die jeweilige Schrittweite. Daraus errechnet sie das eingestellte Dispensiervolumen und bringt es auf dem Display 9 zur Anzeige.

Bei der Anwendung der Pipette 1 wird zunächst eine Spritze 3 mit einer vom Anwender gewählten Spritzengröße lösbar mit der Pipette 1 verbunden, indem sie mit dem ersten Befestigungsabschnitt 16 in die Aufnahme 20 und mit dem zweiten Befestigungsabschnitt 19 in die Aufnahme 24 eingesetzt wird, sodass der Flansch 17 von den ersten Greifhebeln 23 und der Befestigungsabschnitt 19 von den zweiten Greifhebeln 27 gegriffen wird. Diese Situation ist in Fig. 2 gezeigt.

Der Ringsensor 22 tastet die Codierung auf dem Flansch 17 der Spritze 3 ab. Die Elektronik 144 stellt anhand der vom Ringsensor 22 gelieferten Signale fest, dass eine Spritze 3 eingesetzt ist und schaltet das Display 9 ein. Die Elektronik 144 ermittelt anhand der vom Ringsensor 22 und der vom Sensor 132 gelieferten Signale das eingestellte Dispensiervolumen und bringt dieses auf dem Display 9 zur Anzeige. Gegebenenfalls ändert der Anwender mittels des Wahlrades 10 die Einstellung des Dispensiervolumens und das geänderte Dosiervolumen wird vom Display 9 angezeigt.

Zum Aufziehen von Flüssigkeit durch das Loch 14 der Spritze 3 wird der Aufzugshebel 5 aus der Stellung von Fig. 2 nach oben gedrückt.

Am Ende der Aufzugsbewegung nimmt der untere Anschlag 47 das Übertragungsglied 138 mit.

Bevor das eingestellte Dosiervolumen schrittweise abgegeben werden kann, muss ein Umkehrhub ausgeführt werden. Dies geschieht durch Betätigen des Bedienknopfes 8. Hierbei treffen die Schenkel 68, 69 auf die Steuerungsarme 141, 142 und verlagern das Übertragungsglied 138 nach unten. Das Übertragungsglied 138 nimmt über den unteren Anschlag 47 die Zahnstange 28 mit. Durch diesen Umkehrhub wird die Lose aus dem System entfernt.

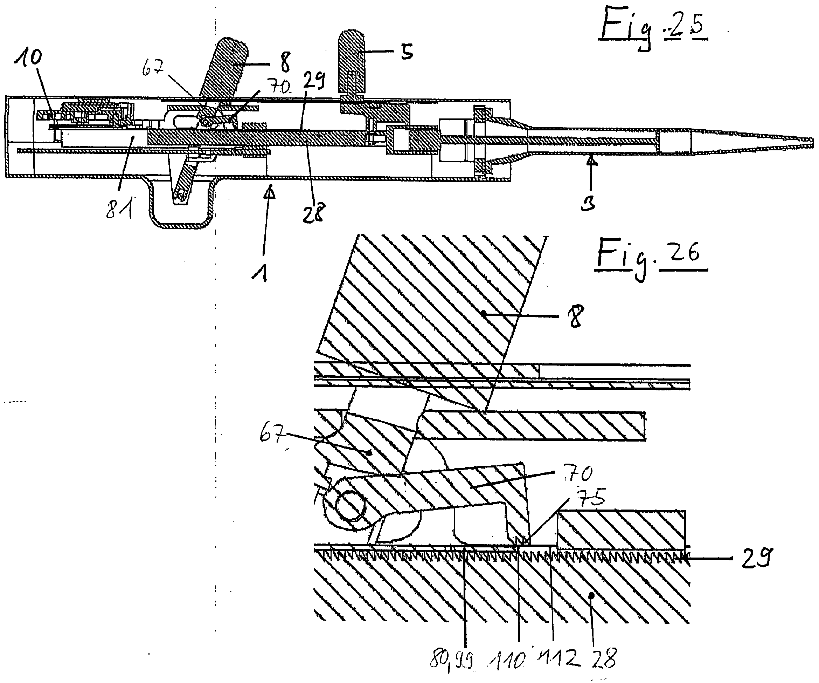

Danach kann die aufgezogene Flüssigkeitsmenge in kleinen Schritten abgegeben werden, indem der Bedienknopf 8 wiederholt entgegen der Wirkung der Federeinrichtung nach unten gedrückt wird. Hierbei drückt die weitere Federeinrichtung die Klinke 70 mit dem Klinkenzahn 75 gegen die Abdeckung, bis der Klinkenzahn 75 das untere Ende der Abhaltefläche 109 erreicht. Diese Situation ist in Fig. 25 und 26 gezeigt.

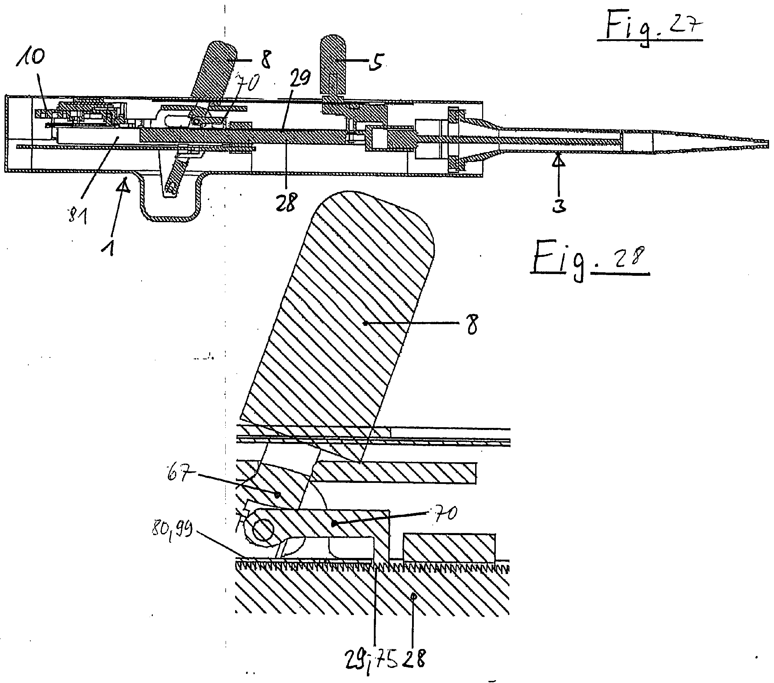

Nach dem Überschreiten der Einfallkante 110 fällt die Klinke 70 mit dem Klinkenzahn 75 in die Zahnung 29 der Zahnstange 28 ein und nimmt die Zahnstange 28 beim weiteren Schwenken des Dosierhebels 67 nach unten ein Stück mit. Diese Situation ist in Fig. 27 und 28 gezeigt. Hierbei hängt die Verlagerung der Zahnstange 28 bei jedem Schwenken des Dosierhebels 67 bis zum Erreichen eines unteren Anschlages von der mittels des Wahlrades 10 eingestellten Position der Abdeckung 80 ab. Nach Entlastung des Bedienknopfes 8 wird er von der Federeinrichtung nach oben gedrückt und ein weiterer Dosierschritt kann ausgeführt werden.

Das Dispensieren ohne Nachbefüllen der Spritze 3 ist solange möglich, bis die in der Spritze 3 verbliebene restliche Flüssigkeitsmenge kleiner als die eingestellte Dosiermenge ist. Dann sorgt die Resthubsperre dafür, dass der Klinkenzahn 75 nicht mehr in die Zahnung 29 einfallen kann, indem der Nocken 133 die Federzunge 112 betätigt, die die Klinke 70 von der Zahnstange wegschwenkt.

Restliche Flüssigkeit in der Spritze 3 kann durch Verlagern des Aufzugshebels 5 nach unten abgegeben werden. Danach kann die Spritze 3 von der Pipette 1 getrennt werden. Hierfür betätigt der Anwender die ersten Greifhebel 23, die über Vorsprünge an der Innenseite auf die zweiten Griffhebel 27 wirken, wie in der EP 0 656 229 B1 und der US 5,620,660 A beschrieben.

Vorzugsweise weist eine erfindungsgemäße Pipette anstatt unmittelbar manuell betätigbarer Greifhebel 23, 27 erste und zweite Mittel zum lösbaren Halten der Spritze auf, die so ausgebildet sind, wie in der EP 2 033 712 A1 und US 2009/139351 A1 beschrieben, deren Inhalt hiermit in die vorliegende Anmeldung aufgenommen wird. Bei der Pipette gemäß den beiden vorgenannten Druckschriften wird die Spritze nach dem Entleeren durch eine weitere Betätigung des Dosierhebels von der Pipette gelöst. Hierfür weist die Pipette ein Getriebe auf, welches vom Dosierhebel gesteuert wird und auf die ersten und zweiten Mittel zum Halten der Spritze einwirkt, um die Spritze von der Pipette zu lösen. Die ersten und zweiten Mittel zum lösbaren Halten sind gemäß einer Ausgestaltung dieser Pipette als Greifhebel ausgebildet, die in das Gehäuse integriert sind und nicht unmittelbar von außen betätigbar sind.

Gemäß den Fig. 27 und 28 fällt die Klinke 70 optimal in die Zahnung ein, d.h. jeder Klinkenzahn greift genau in ein Tal zwischen zwei Zähnen der Zahnung ein.

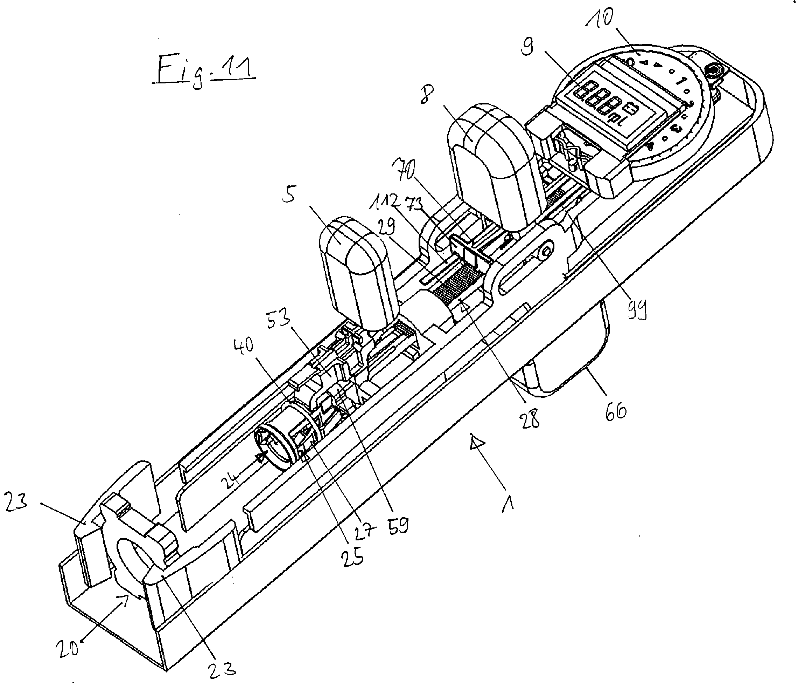

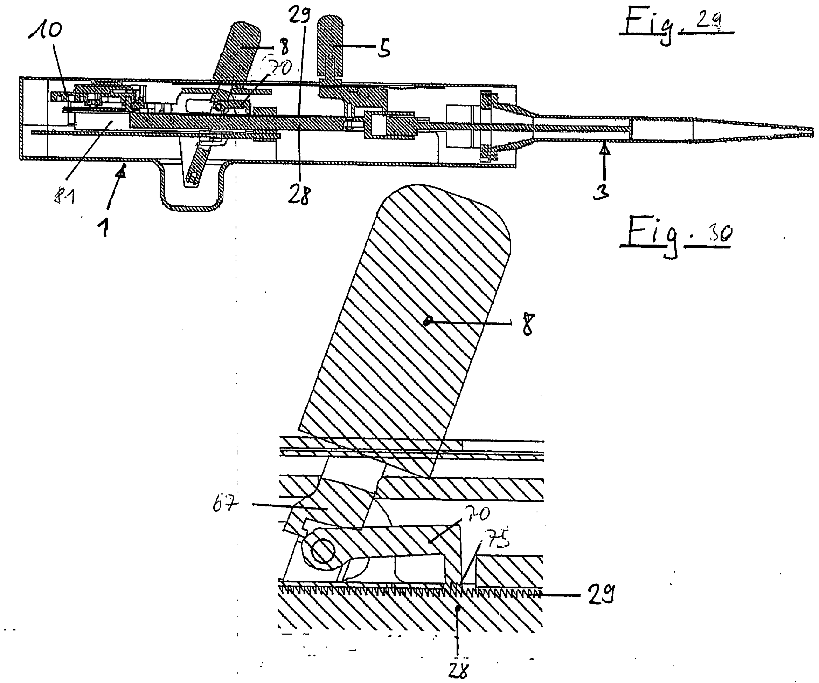

Gemäß Fig. 29 und 30 sind Klinkenzähne 75 ungünstig auf die Zahnung 29 ausgerichtet, sodass sie beim Schwenken des Dosierhebels 67 in der falschen Position einfallen oder über die Zahnung 29 hinwegrutschen können. Durch Justieren der Abdeckung 80 in der anhand von Fig. 15 bis 17 beschriebenen Weise kann dies überwunden werden, sodass die Klinke 70 in die Zahnung 29 einfällt, wie in den Fig. 27 und 28 gezeigt. Die Justierung ist durch ein Loch im Gehäuse 2 oder nach Abnehmen der vorderen Gehäusehälfte durch Ansetzen eines Werkzeuges an das Exzenterteil 122 möglich (vgl. Fig. 11).

### Bezugszeichenliste

- 1: Pipette
- 2: Gehäuse
- 3: Spritze
- 4: Schlitz
- 5: Aufzugshebel
- 6: Schlitz
- 7: Schlitz
- 8: Bedienknopf
- 9: Display
- 10: Wahlrad
- 11: Zylinder
- 12: Kolben
- 13: konischer Abschnitt
- 14: Loch
- 15: zylindrischer Abschnitt
- 16: erster Befestigungsabschnitt
- 17: Flansch
- 18: Kolbenstange
- 19: zweiter Befestigungsabschnitt
- 20: erste Aufnahme
- 21: erste Öffnung
- 22: Ringsensor
- 23: erster Greifhebel
- 24: zweite Aufnahme
- 25: Aufnahmekörper
- 26: zweite Öffnung
- 27: zweiter Greifhebel
- 28: Zahnstange
- 29: Zahnung
- 30: Ringelement
- 31, 32: Strebe
- 33: Rastvorsprung
- 34: seitliche Öffnung (Langloch)
- 35: weitere seitliche Öffnung (weiteres Langloch)
- 36: Lagerauge
- 37: Achse (Stift)
- 38: weiterer zweiter Greifhebel
- 39: weiterer Rastvorsprung
- 40: O-Ring
- 41: Vertiefung
- 42, 43: seitlicher Schenkel
- 44: Basis
- 45: mittlerer Schenkel
- 46: Rippe
- 47: unterer Anschlag
- 48: Zunge
- 49: Längsschlitz
- 50: Bohrung
- 51: Aufnahme
- 52: Führungsrippe
- 53: Aufzugshebelhalterung
- 54: Sockel
- 55: Arm
- 56: Vertiefung
- 57, 58: Rastelement
- 59: Schieber
- 60, 61: Rampe
- 62: Aufzugshebelträger
- 63: Gleitplatte
- 64: Pfosten
- 65: Schwenklager
- 66: Ausbuchtung
- 67: Dosierhebel
- 68: Schenkel
- 69: Schenkel
- 70: Klinke
- 71: Gleitlager
- 72: Klinkenarm
- 73: Zahnträger
- 74: Schwenkachse
- 75: Klinkenzahn
- 76: Zahnschneide
- 77: Klinkendurchbruch
- 78, 79: Zahnflanke
- 80: Abdeckung
- 81: erstes Abdeckungsteil
- 82: Seitenteile
- 83: Seitenteile
- 84: Überbrückungsteil
- 85: Flügelteil
- 86: Flügelteil
- 87: Stift
- 88: Nutenstein
- 89: Lagerbohrung
- 90: vordere Vertiefung
- 91: hintere Vertiefung
- 92: Bohrungsrand
- 93: erste Aussparung
- 94: Führungshaken
- 95: Führungshaken
- 96: Längsnut
- 97: Kanal
- 98: weiterer Längsschlitz
- 99: zweites Abdeckungsteil
- 100: Kopfabschnitt
- 101: Mittelabschnitt
- 102: Mittelabschnitt
- 103: gegenläufige Abwinklung
- 104: gegenläufige Abwinklung
- 105: Fußabschnitt
- 106: erster Verbindungsabschnitt
- 107: mittlerer Streifenabschnitt
- 108: vorstehende Abwinklung
- 109: Abhaltefläche
- 110: Einfallkante
- 111: zweiter Verbindungsabschnitt
- 112: Federzunge
- 113: Loch
- 114: Ausstanzung
- 115: Zunge
- 116: Zunge
- 117: Zunge
- 118: Zunge
- 119: Freiraum
- 120: Freiraum
- 121: Exzenteraufnahme
- 122: Exzenterteil
- 123: Welle
- 124: Exzenter
- 125: Werkzeugangriff
- 126: Scheibe
- 127: Rastvorsprung
- 128: zweite Aussparung
- 129: Führungsschlitz
- 130: Lagerloch
- 131: Führungskurve
- 132: weiterer Sensor
- 133: Nocken
- 134: axiale Bohrung
- 135: Einstellschraube
- 136: Mutter
- 137: Schraubenfeder
- 138: Übertragungsglied
- 139: Übertragungsabschnitt
- 140: unteres Übertragungsgliedende
- 141: Steuerungsarm
- 142: Steuerungsarm
- 143: Leiterplatte
- 144: Elektronik
- 145: Akku

## Patentansprüche

1. Dispenser zum Betätigen einer Spritze für das Aufnehmen und schrittweise Abgeben von Flüssigkeit mit
• einem stangenförmigen Gehäuse (2),
• einer ersten Aufnahme (20) mit einer ersten Öffnung (21) am unteren Ende des Gehäuses (2) zum Einsetzen eines ersten Befestigungsabschnitts (16) am oberen Rand eines Zylinders (11) der Spritze (3),
• einem im Gehäuse (2) angeordneten Aufnahmekörper (25) mit einer zweiten Aufnahme (24) und einer zweiten Öffnung (26) am unteren Ende zum Einsetzen eines zweiten Befestigungsabschnitts an einem Kolben (12) der Spritze (3),
• ersten Mitteln zum lösbaren Halten des ersten Befestigungsabschnitts (16) in der ersten Aufnahme (20),
• zweiten Mitteln zum lösbaren Halten des zweiten Befestigungsabschnitts (19) in der zweiten Aufnahme (24),
• ersten Mitteln zum Verlagern (5) des Aufnahmekörpers (25) im Gehäuse (2) nach oben,
• zweiten Mitteln zum schrittweisen Verlagern des Aufnahmekörpers (25) im Gehäuse (2) nach unten, umfassend
• ein aus dem Gehäuse (2) herausragendes Betätigungselement (67) zum Durchführen einzelner Schritte,
• eine im Gehäuse (2) angeordnete und mit dem Aufnahmekörper (25) verbundene Zahnstange (28) mit einer Zahnung (29) und
• eine im Gehäuse (2) am Betätigungselement (67) schwenkbar gelagerte Klinke (70), die bei einer Verlagerung des Betätigungselements (67) nach unten mit der Zahnung (29) der Zahnstange (28) in Eingriff kommt und diese mitnimmt und bei Verlagerung des Betätigungselements (67) nach oben außer Eingriff mit der Zahnung (29) kommt,
**dadurch gekennzeichnet, dass**
• die Zahnstange (28) und der Aufnahmekörper (25) einteilig aus Kunststoff hergestellt sind und
• die zweiten Mittel zum lösbaren Halten mindestens einen in einer seitlichen Öffnung (34) des Aufnahmekörpers (25) angeordneten und einteilig mit dem Aufnahmekörper (25) aus elastischem Kunststoff hergestellten zweiten Greifhebel (27) aufweisen.

2. Dispenser nach Anspruch 1, bei dem der Aufnahmekörper (25) hohlzylindrisch und am Boden einteilig mit dem unteren Ende der Zahnstange (28) verbunden ist.

3. Dispenser nach Anspruch 1 oder 2, bei dem der Aufnahmekörper (25) ein Käfig ist, der am unteren Ende ein Ringelement (30) und von dem Ringelement ausgehend parallel nach oben erstreckte Streben (31, 32) aufweist, die am oberen Ende mit dem unteren Ende der Zahnstange (28) verbunden sind.

4. Dispenser nach Anspruch 3, bei dem der Käfig eine vordere Strebe (31) auf der Seite der Zahnung (29) der Zahnstange (28) und eine hintere Strebe (32) auf der von der Zahnung (29) abgewandten Seite der Zahnstange (28) aufweist.

5. Dispenser nach einem der Ansprüche 1 bis 4, bei dem der Aufnahmekörper (25) an der zweiten Öffnung (26) trichterförmig nach unten erweitert ist.

6. Dispenser nach einem der Ansprüche 1 bis 5, bei dem der zweite Greifhebel (27) einarmig ist, an einem Ende mit dem Aufnahmekörper (25) verbunden ist, sich parallel zur Zahnstange (28) erstreckt und in einem Abstand von seinem mit dem Aufnahmekörper (25) verbundenen Ende einen nach innen vorstehenden Rastvorsprung (33) aufweist.

7. Dispenser nach einem der Ansprüche 1 bis 6, bei dem der Aufnahmekörper (25) eine weitere seitliche Öffnung (35) aufweist, an beiden Längsseiten der weiteren seitlichen Öffnung (35) Lageraugen (36) nach außen von dem Aufnahmekörper vorstehen, ein weiterer zweiter Greifhebel (27) an einem Ende auf einer Welle oder Achse (37) in die weitere seitliche Öffnung (35) hinein schwenkbar an den Lageraugen gelagert ist, der in einem Abstand von der Welle oder Achse (37) einen innen vorstehenden Rastvorsprung (39) aufweist.

8. Dispenser nach Anspruch 3 und 6 oder 7, bei dem der zweite Greifhebel (27) in einem Langloch (34) zwischen den beiden Streben (31, 32) angeordnet ist und/oder bei dem der weitere zweite Greifhebel in einem weiteren Langloch (35) in einer Strebe (32) angeordnet ist.

9. Dispenser nach einem der Ansprüche 6 bis 8, bei dem der zweite Greifhebel (27) an seinem unteren Ende einteilig mit den Aufnahmekörper (25) verbunden ist und/oder bei dem der weitere zweite Greifhebel (27) an seinem unteren Ende auf der Welle oder Achse (37) gelagert ist.

10. Dispenser nach einem der Ansprüche 1 bis 9, bei dem unten auf der Zahnstange (28) und/oder oben auf dem Aufnahmekörper (25) ein Schieber (59) in Längsrichtung der Zahnstange (28) verschieblich angeordnet ist, der auf mindestens einer Seite eine Rampe (60, 61) aufweist, deren Abstand von der Zahnstange (28) nach oben zunimmt, wobei der Schieber nach unten verlagerbar ist, sodass die Rampe (60, 61) das obere Ende eines zweiten Greifhebels (27) innen kontaktiert und bei weiterer Verlagerung des Schiebers (59) nach unten den zweiten Greifhebel (27) nach außen schwenkt.

11. Dispenser nach einem der Ansprüche 1 bis 10, bei dem sich die Zahnung (29) über die gesamte Breite der Zahnstange (28) erstreckt und/oder bei dem die Klinke (70) mindestens einen Klinkenzahn (75) aufweist, der sich über weniger als die gesamte Breite der Zahnung (29) erstreckt.

12. Dispenser nach Anspruch 11, bei dem der Klinkenzahn (75) einen Klinkendurchbruch (77) aufweist, der sich senkrecht zur Schwenkachse (74) der Klinke (70) erstreckt und auf der Seite der Zahnschneide (76) und zu den beiden Zahnflanken (78, 79) jedes Klinkenzahns (75) hin geöffnet ist.

13. Dispenser nach einem der Ansprüche 1 bis 12, bei dem das erste Mittel zum Verlagern (5) ein aus dem Gehäuse herausragender Aufzugshebel (5) ist, der mit einer an der Zahnstange (28) und/oder dem Aufnahmekörper (25) fixierten Aufzugshebelhalterung (53) verbunden ist.

14. Dispenser nach einem der Ansprüche 1 bis 13 bei dem die Zahnstange (28) oben einen Längsschlitz (49) für einen Nocken (133) einer Resthubsperre aufweist.

15. Dispenser nach einem der Ansprüche 1 bis 14, bei dem die Zahnstange (28) einen T-förmigen und/oder einen U-förmigen Querschnitt aufweist, wobei die Zahnung (29) an der Außenseite des Querbalkens des T-Querschnitts und/oder der Basis des U-Querschnitts angeordnet ist.

16. Dispenser nach einem der Ansprüche 1 bis 15, bei dem die Zahnstange (28) und der Aufnahmekörper (25) einteilig aus Kunststoff spritzgegossen und/oder bei dem die Klinke (70) einteilig aus Kunststoff spritzgegossen ist und/oder bei dem die Zahnstange (28) und/oder der Aufnahmekörper (25) und/oder die Klinke (70) aus PEEK oder aus einem anderen hochfesten Kunststoff hergestellt sind.

## Claims

1. A dispenser for actuating an injection syringe for receiving and gradually dispensing liquid with
• a rod-shaped housing (2),
• a first reception (20) with a first opening (21) at the lower end of the housing (2) for inserting a first fastening section (16) on the upper edge of a cylinder (11) of the injection syringe (3),
• a receiving body (25) with a second reception (24) and a second opening (26) at the lower end for inserting a second fastening section on a piston (12) of the injection syringe (3),
• first means for releaseably holding the first fastening section (16) in the first reception (20),
• second means for releaseably holding the second fastening section (19) in the second reception (24),
• first means for displacing (5) the receiving body (25) upwards in the housing (2),
• second means for gradually displacing the receiving body (25) downwards in the housing (2), comprising,
• an actuating member (67) projecting from the housing (2), for performing individual steps,
• a toothed rack (28) with a set of teeth (29) arranged in the housing (2) and connected to the receiving body (25), and
• a pivotably mounted latch (70) on the actuating member (67) in the housing (2), which, when the actuating member (67) is displaced downwards, engages the teeth (29) of the toothed rack (28) and takes said set of teeth with it and, when the actuating member (67) is displaced upwards, upwardly disengages the set of teeth (29),
**characterised in that**
• the toothed rack (28) and the receiving body (25) are manufactured out of plastic as a single piece and
• the second means for releaseably holding have at least one second lever handle (27), which is arranged in a side opening (34) of the receiving body (25) and manufactured out of elastic plastic as a single piece with the receiving body (25).

2. The dispenser according to claim 1, in which the receiving body (25) is a hollow cylinder and is integrally connected at its base with the lower end of the toothed rack (28).

3. The dispenser according to claim 1 or 2, in which the receiving body (25) is a cage which has a ring member (30) at the lower end and, proceeding from the ring member, struts (31, 32) extended upwards in parallel, which, at the upper end, are connected to the lower end of the toothed rack (28).

4. The dispenser according to claim 3, in which the cage has a front strut (31) on the side of the teeth (29) of the toothed rack (28) and a rear strut (32) on the side of the toothed rack (28) facing away from the teeth (29).

5. The dispenser according to one of claims 1 to 4, in which the receiving body (25) is extended in a funnel-shape at the second opening (26).

6. The dispenser according to one of claims 1 to 5, in which the second lever handle (27) is one-armed and connected to the receiving body (25) at one end, extends parallel to the toothed rack (28) and has a rest projection (33) protruding inwards at a distance from its end which is connected to the receiving body (25).

7. The dispenser according to one of claims 1 to 6, in which the receiving body (25) has an additional side opening (35), bearing eyes (36) protrude outwards from the receiving body on both longitudinal sides of the additional side opening (35), an additional second lever handle (27) on one end of a shaft or axle (37) is pivotably mounted on the bearing eyes into the additional side opening (35), which has a rest projection (39) protruding inwards at a distance from the shaft or axis (37).

8. The dispenser according to claim 3 and 6 or 7, in which the second lever handle (27) is arranged in an oblong hole (34) between the two struts (31, 32) and/or in which the additional second lever handle is arranged in an additional oblong hole (35) in one strut (32).

9. The dispenser according to one of claims 6 to 8, in which the second lever handle (27) is connected at its lower end to the receiving body (25) as a single piece and/or in which the additional second lever handle (27) is mounted at its lower end on the shaft or axle (37).

10. The dispenser according to one of claims 1 to 9, in which a slider (59) is displaceably arranged at the bottom on the toothed rack (28) and/or at the top on the receiving body (25) in longitudinal direction of the toothed rack (28), which has one ramp (60, 61) on at least one side, of which the distance from the toothed rack (28) increases towards the top, wherein the slider can be displaced downwards, so that the ramp (60, 61) comes into contact inside with the upper end of a second lever handle (27) and, when the slider (59) is further displaced downwards, pivots the lever handle outwards.

11. The dispenser according to one of claims 1 to 10, in which the teeth (29) extend over the whole width of the toothed rack (28) and/or in which a latch (70) has at least one latch tooth (75) which extends over less than the whole width of the teeth (29).

12. The dispenser according to claim 11, in which the latch tooth (75) has a latch aperture (77) which extends vertically to the pivot axis (74) of the latch (70) and is open on the side of the tooth cutting-edge (76) and towards both tooth flanks (78, 79) of each latch tooth (75).

13. The dispenser according to one of claims 1 to 12, in which the first means for displacing (5) is a lifting lever (5) projecting from the housing, which is connected to a fixed lifting lever mount (53) on the toothed rack (28) and/or on the receiving body (25).

14. The dispenser according to one of claims 1 to 13, in which the toothed rack (28) has at the top a longitudinal slot (49) for a cam (133) of a residual stroke lock.

15. The dispenser according to one of claims 1 to 14, in which the toothed rack (28) has a T-shaped and/or U-shaped cross-section, wherein the set of teeth (29) is arranged on the outside of the crossbar of the T-shaped cross-section and/or the base of the U-shaped cross-section.

16. The dispenser according to one of claims 1 to 15, in which the toothed rack (28) and the receiving body (25) is injection moulded out of plastic as a single piece and/or in which the latch (70) is injection moulded out of plastic as a single piece and/or in which the toothed rack (28) and/or the receiving body (25) and/or the latch (70) are made out of PEEK or out of another high-strength plastic.

## Revendications

1. Distributeur destiné à actionner une seringue pour le prélèvement et la délivrance progressive de liquide, comprenant:
• un logement (2) en forme de tige,
• une première admission (20) avec une première ouverture (21) à l'extrémité inférieure du logement (2), pour l'insertion d'une première partie de fixation (16) sur le bord supérieur d'un cylindre (11) de la seringue (3),
• un corps d'admission (25) disposé dans le logement (2) avec une deuxième admission (24) et une deuxième ouverture (26) à l'extrémité inférieure pour l'insertion d'une deuxième partie de fixation sur un piston (12) de la seringue (3),
• des premiers moyens destinés à maintenir la première partie de fixation (16) de façon amovible dans la première admission (20),
• des deuxièmes moyens destinés à maintenir la deuxième partie de fixation (19) de façon amovible dans la deuxième admission (24),
• des premiers moyens (5) destinés à déplacer le corps d'admission (25) vers le haut dans le logement (2),
• des deuxièmes moyens destinés à déplacer progressivement le corps d'admission (25) vers le bas dans le logement (2), comportant
• un élément d'actionnement (67) faisant saillie hors du logement (2), pour l'exécution de différentes étapes,
• une crémaillère (28) avec une denture (29), disposée dans le logement (2) et reliée au corps d'admission (25), et
• un cliquet (70) monté de façon pivotante sur l'élément d'actionnement (67) dans le logement (2), lequel vient en prise avec la denture (29) de la crémaillère (28) lors du déplacement de l'élément d'actionnement (67) vers le bas, tout en entraînant celle-ci, et se dégage de la denture (29) lors d'un déplacement de l'élément d'actionnement (67) vers le haut,
**caractérisé en ce que**
• la crémaillère (28) et le corps d'admission (25) sont fabriqués d'une seule pièce en plastique, et **en ce que**
• les deuxièmes moyens destinés au maintien amovible présentent au moins un deuxième levier de préhension (27) fabriqué en plastique élastique d'une seule pièce avec le corps d'admission (25) et disposé dans une ouverture latérale (34) du corps d'admission (25).

2. Distributeur selon la revendication 1, dans lequel le corps d'admission (25) est un cylindre creux, relié à la base d'un seul tenant à l'extrémité inférieure de la crémaillère (28).

3. Distributeur selon la revendication 1 ou 2, dans lequel le corps d'admission (25) est une cage présentant un élément annulaire (30) à l'extrémité inférieure et des barreaux (31, 32) s'étendant parallèlement vers le haut à partir de l'élément annulaire, lesquels sont reliés par leur extrémité supérieure à l'extrémité inférieure de la crémaillère (28).

4. Distributeur selon la revendication 3, dans lequel la cage présente un barreau avant (31) sur le côté de la denture (29) de la crémaillère (28) et un barreau arrière (32) sur le côté de la crémaillère (28) qui est détourné de la denture (29).

5. Distributeur selon l'une des revendications 1 à 4, dans lequel le corps d'admission (25) s'élargit de façon conique vers le bas au niveau de la deuxième ouverture (26).

6. Distributeur selon l'une des revendications 1 à 5, dans lequel le deuxième levier de préhension (27) présente un seul bras, est relié par une extrémité au corps d'admission (25), s'étend parallèlement à la crémaillère (28) et présente une saillie d'encliquetage (33) faisant saillie vers l'intérieur, à une distance de son extrémité reliée au corps d'admission (25).

7. Distributeur selon l'une des revendications 1 à 6, dans lequel le corps d'admission (25) présente une autre ouverture latérale (35), des oeillets de montage (36) font saillie vers l'extérieur à partir du corps d'admission, sur les deux côtés longitudinaux de l'autre ouverture latérale (35), un autre deuxième levier de préhension (27) est monté à une extrémité sur un arbre ou axe (37) sur les oeillets de montage de façon à pouvoir pivoter vers l'intérieur de l'autre ouverture latérale (35), lequel présente une saillie d'encliquetage (39) faisant saillie vers l'intérieur, à une distance de l'arbre ou axe (37).

8. Distributeur selon les revendications 3 et 6 ou 7, dans lequel le deuxième levier de préhension (27) est disposé dans un trou oblong (34) situé entre les deux barreaux (31, 32), et/ou dans lequel l'autre deuxième levier de préhension est disposé dans un autre trou oblong (35) situé dans un barreau (32).

9. Distributeur selon l'une des revendications 6 à 8, dans lequel le deuxième levier de préhension (27) est relié par son extrémité inférieure au corps d'admission (25), et/ou dans lequel l'autre deuxième levier de préhension (27) est monté avec son extrémité inférieure sur l'arbre ou axe (37).

10. Distributeur selon l'une des revendications 1 à 9, dans lequel un poussoir (59) est disposé en bas sur la crémaillère (28) et/ou en haut sur le corps d'admission (25), de façon à pouvoir coulisser dans le sens longitudinal de la crémaillère (28), lequel présente une rampe (60, 61) sur au moins un côté, dont l'écart par rapport à la crémaillère (28) augmente vers le haut, le poussoir pouvant être déplacé vers le bas de manière à ce que la rampe (60, 61) touche l'extrémité supérieure d'un deuxième levier de préhension (27) à l'intérieur, et fait pivoter le deuxième levier de préhension (27) vers l'extérieur lors d'un déplacement supplémentaire du poussoir (59) vers le bas.

11. Distributeur selon l'une des revendications 1 à 10, dans lequel la denture (29) s'étend sur toute la largeur de la crémaillère (28), et/ou dans lequel le cliquet (70) présente au moins une dent de cliquet (75) s'étendant sur moins de la totalité de la largeur de la denture (29).

12. Distributeur selon la revendication 11, dans lequel la dent de cliquet (75) présente une percée de cliquet (77) s'étendant perpendiculairement à l'axe de pivotement (74) du cliquet (70) et ouverte vers le côté du tranchant de dent (76) ainsi que vers les deux flancs de dent (78, 79) de chaque dent de cliquet (75).

13. Distributeur selon l'une des revendications 1 à 12, dans lequel le premier moyen destiné au déplacement (5) est un levier de relèvement (5) faisant saillie hors du logement, lequel est relié à un support de levier de relèvement (53) fixé à la crémaillère (28) et/ou au corps d'admission (25).

14. Distributeur selon l'une des revendications 1 à 13, dans lequel la crémaillère (28) présente une fente longitudinale (49) sur le haut, pour une came (133) d'un verrou de course résiduelle.

15. Distributeur selon l'une des revendications 1 à 14, dans lequel la crémaillère (28) présente une section transversale en forme de T et/ou en forme de U, la denture (29) étant disposée sur le côté extérieur de la barre transversale de la section en T et/ou de la base de la section en U.

16. Distributeur selon l'une des revendications 1 à 15, dans lequel la denture (28) et le corps d'admission (25) sont moulés par injection à partir d'une seule pièce en plastique, et/ou dans lequel le cliquet (70) est moulé par injection à partir d'une seule pièce en plastique, et/ou dans lequel la crémaillère (28) et/ou le corps d'admission (25) et/ou le cliquet (70) sont fabriqués en PEEK ou un autre plastique hautement résistant.
